# EUROPÄISCHE PATENTSCHRIFT

(11) **EP 1 365 840 B1**
(45) Veröffentlichungstag und Bekanntmachung des Hinweises auf die Patenterteilung: **13.10.2010**
(21) Anmeldenummer: 02750510.6
(22) Anmeldetag: 05.03.2002
(51) Int. Cl.: A61N 5/06

(54) **BESTRAHLUNGSANORDNUNG-FÜR THERAPEUTISCHE ZWECKE**
IRRADIATING DEVICE FOR THERAPEUTIC PURPOSES
SYSTEME D'IRRADIATION UTILISE A DES FINS THERAPEUTIQUES

(30) Priorität: 08.03.2001 DE 20109899 U; 10.05.2001 DE 10123926
(43) Veröffentlichungstag der Anmeldung: 03.12.2003
(73) Patentinhaber: Spectrometrix Optoelectronic Systems GmbH, 12489 Berlin (DE)
(72) Erfinder: WILKENS, Jan, Henrik, 66242 Homburg (DE); STIRNER, Rolf, 15831 Mahlow-Waldblick (DE)
(74) Vertreter: Patentanwälte Bressel und Partner
(86) Internationale Anmeldenummer: PCT/DE2002/000778
(87) Internationale Veröffentlichungsnummer: WO 2002/072200

(56) Entgegenhaltungen:
- EP-A- 0 726 083
- EP-A- 0 765 674
- EP-B- 0 565 331
- WO-A-00/02491
- WO-A-02/32505
- WO-A-98/23329
- WO-A1-02/32505
- DE-A- 19 838 304
- DE-A- 19 852 524
- US-A- 5 798 523
- US-A- 5 968 034
- SIGURDSSON ET AL.: "Phototherapy of Acne Vulgaris with Visible Light" DERMATOLOGY, Bd. 194, 1997, Seiten 256-260, XP000863088

## Beschreibung

Die Erfindung betrifft eine Bestrahlungsanordnung für therapeutische Zwecke, insbesondere zur akuten oder chronischen Behandlung von ganz oder teilweise zellvermittelten Entzündungen der Haut, der Bindegewebe und der inneren Organe, viralen und anderen infektösen Erkrankungen wie HIV oder Prioneninfektionen, Pilzinfektionen der Haut und der Schleimhäute, bakteriellen Erkrankungen der Haut und der Schleimhäute sowie Hand- und Analekzemen.

Therapeutische Bestrahlungsanordnungen sind insbesondere im Bereich der Phototherapie von Hauterkrankungen seit langem bekannt. Je nach Anwendungsgebiet wird dabei ein Patient im Wellenlängenbereich von 315-1500 nm bestrahlt. Insbesondere der Wellenlängenbereich zwischen 315-340 nm (UV-A2) kann nachweislich zu einem erhöhten Krebsrisiko führen, so daß insbesondere bei der Behandlung von atopischen Ekzemen die UV-A1-Therapie (340-400 nm) zur Anwendung kommt.

Die Chemo-Phototherapie als Oberbegriff umfaßt allgemein die Verwendung von optischer Strahlung zur Erzielung von therapeutischen Effekten. Ein Untergebiet der Chemo-Phototherapie ist die sogenannte photodynamische Therapie PDT. Zwei Hauptanwendungsgebiete der PDT sind die Krebsbehandlung und die Behandlung von ganz oder teilweise zellvermittelten Hautentzündungen. Gemeinsames Merkmal der PDT ist die Erzeugung von reaktiven Sauerstoff-Spezies. Hierzu regt die optische Strahlung inner- oder äußerlich verabreichte Farbstoffmoleküle an, die dann in einen angeregten Zustand überführt werden. Durch Wechselwirkung mit vorhandenen Sauerstoffmolekülen werden durch Energieübertragung reaktive Sauerstoff-Spezies gebildet, die dann die Zelle schädigen oder gar zerstören.

Bei der Krebstherapie mit PDT, wo die Tumorzellen zerstört werden sollen, sind zwei Anwendungsgebiete zu unterscheiden. Der Hauptanwendungsfall ist die Tumorbehandlung innerer Organe. Hierzu wird endoskopisch über eine Glasfaser die optische Strahlung eines Laser zu dem Tumor geleitet und dieser punktuell bestrahlt. Des weiteren werden dem Patienten Photosensibilisatoren gespritzt. Dabei tritt das Problem auf, daß das Tumorgewebe erheblich schlechter durchblutet ist, so daß auch der Sauerstoffgehalt sehr niedrig ist, was aber auch die Umwandlung in reaktive Sauerstoff-Spezies begrenzt. Daher ist es bei der Tumorbehandlung innerer Organe mit PDT bekannt, dem Patienten inspiratorisch erhöht Sauerstoff zuzuführen, so daß der Sauerstoffgehalt im Tumorgewebe erhöht wird und vermehrt reaktive Sauerstoff-Spezies gebildet werden können. Aufgrund des erhöhten Sauerstoffverbrauches ist es bekannt, die Bestrahlungsquelle im Pulsbetrieb zu betreiben, so daß in den Pulspausen Sauerstoff nachdiffundieren kann. Das zweite Anwendungsgebiet in der Krebstherapie mit PDT ist die Behandlung von äußeren Tumoren wie insbesondere Hautkrebs, wo aufgrund des vorhandenen Sauerstoffs in der Umgebung auf eine zusätzliche Sauerstoffzugabe verzichtet wird.

Bei der Behandlung von ganz oder teilweise zellvermittelten Hautentzündungen handelt es sich im Gegensatz zu Tumoren stets um großflächige Entzündungen, so daß hier flächenhafte Strahlungsquellen zur Anwendung kommen, die Behandlungsflächen von beispielsweise 5 cm² bis 2 m² simultan bestrahlen. Ein weiterer Unterschied zu den Tumoren ist die erhöhte Durchblutung von Entzündungen, die bereits äußerlich durch die vorhandene Rötung im Entzündungsbereich gut erkennbar ist. Des weiteren findet keine exogene Sensibilisatorapplikation statt, so daß selbst unter der Annahme einer phototherapeutisch induzierten Beteiligung von Singulett-Sauerstoff, z.B. über die photodynamische Wirkung endogener Porphyrine, davon auszugehen ist, daß selbst bei einer erheblichen Verminderung der Sauerstoffkonzentration in der Haut es nur zu einer unwesentlichen Verminderung der Triplet-Effizienz kommt. Darüber hinaus liegt die maximale Konzentration endogener Photosensibilisatoren um mehrere Größenordnungen unter der Konzentration, die nach topischer und/oder systemischer Applikation erreichbar ist. Die erwähnte gute Hautdurchblutung in Verbindung mit der geringen Sensibilisatorkonzentration führte dazu, daß es bisher nicht zu einer synchronen Foto/Sauerstofftherapie bei den zellvermittelten Erkrankungen gekommen ist.

Das überwiegend in der Behandlung von ganz oder teilweise zellvermittelten Hauterkrankungen eingesetzte Verfahren der PDT ist die hochdosierte UV-A1-Therapie im Wellenlängenbereich von 340-400 nm. Dabei ist zum einen die Abgabe hoher Dosen von beispielsweise 130 J/cm² und zum anderen die Bestrahlungsstärke von mehr als 60 mW/cm² erforderlich, um ausreichende therapeutische Effekte zu bewirken. Trotzdem bleibt bei ca. 20 - 30 % der behandelten Patienten die UV-A1-Therapie wirkungslos.

Es ist weiter bekannt, Akne, eine aufgrund von Bakterienwachstum in verstopften Follikeln talgdrüsenreicher Hautbezirke mit Verhornungsstörungen hervorgerufene Hauterkrankung, mit blauem Licht im Bereich von 400 - 440 nm ohne wesentliche UVA-Anteile zu behandeln, wobei die Erfolge beschränkt blieben. Hierzu sei auf den Fachartikel "V. Sigurdsson et al. Phototherapy of Acne Vulagris with visible Light, Dermatologie 1997, 194; Bd. 3, 256 -260" mit weiteren Literaturhinweisen verwiesen. Angestoßen wurde diese Form der Therapie, daß Aknefollikel im Rahmen der dermatologischen Untersuchung mit einer sogenannten "woodlamp" rot fluoreszieren. Als Quelle der Fluoreszenz wurde die Speicherung großer Mengen von Porphyrinen im Propionibakterium acne nachgewiesen. (Mc Ginley et al., Facial follicular porphyrin fluorescence. Correlation with age and density of propionibacteriium acnes, Br. J. Dermatol Vol.102., Bd.3, 437-441, 1980). Da Porphyrine ihre Hauptabsorption (Soret-band) um 420 nm haben, war es für Meffert et al. naheliegend, bakterielle Aknefollikel mit blauem Licht zu behandeln. Die langwelligste Absorptionsbande der Porphyrine liegt bei 630 nm mit einer Eindringtiefe von 4 mm, die für eine photodynamische Follikelbehandlung am besten geeignet ist und auch verwendet wird.

Aus der WO 00102491 ist eine derartige Bestrahlungsanordnung zur Aknebehandlung bekannt, die mindestens ein schmalbandiges Spektrum im Bereich von 405 - 440 nm umfaßt. Als alternative oder kumulative Spektralbereiche sind die Wellenlängenintervalle von 610 - 670 nm bzw. 520 - 550 nm angegeben. Zur weiteren Verbesserung des Wirkungsgrades wird vorgeschlagen, die zu bestrahlende Partie mit Sauerstoff anzureichern, indem mit Sauerstoff angereicherte Emulsionen vor oder während der Bestrahlung auf die zu bestrahlende Fläche aufgetragen werden. Die Bestrahlungsstärke liegt dabei zwischen 10 - 500 mW/cm².

Aus der EP 0 565 331 B1 ist eine Vorrichtung zur Behandlung von Gefäßerkrankungen in einem Bereich der Haut bekannt, umfassend ein Gehäuse, mit einer inkohärenten Lichtquelle, montiert in dem Gehäuse und geeignet zum Produzieren von gepulstem Licht für die Behandlung und eine Öffnung in dem Gehäuse, welche einen austretenden Lichtstrahl bestimmt, der auf den Hautbehandlungsbereich gesendet wird, ohne durch ein Kabel aus optischen Fasern zu gehen, und der einen breiteren Strahlungsbereich aufweist als Vorrichtungen mit Kabel aus optischen Fasern, wobei die Vorrichtung ein die niedrigen Frequenzen abschneidendes Filter umfaßt, um die sichtbaren und ultravioletten Teile des Spektrums herauszuschneiden und die inkohärente Lichtquelle einen Ausgangslichtstrahl mit Wellenlängen im Bereich zwischen 300 und 1000 nm produziert. Die Lichtquelle ist elektrisch mit einer Variabel-Impulsbreiten-Erzeugerschaltung verbunden, um einen geregelten Zeitimpuls zu liefern mit einer Breite zwischen 1 und 10 ms, wobei der austretende Lichtstrahl auf der Haut eine Energiedichte zwischen 30 und 100 J/cm² erzeugt, so daß der hinaustretende Lichtstrahl nach Durchgang durch das obengenannte, die niedrigen Frequenzen abschneidende Filter in die Haut so tief wie gewünscht hineindringen kann, ohne die Haut zu verbrennen, um ein unter der Haut und innerhalb des Hautbehandlungsbereiches liegendes Blutgefäß zu erwärmen und im Blutgefäß Blutkoagulation zu verursachen. Die dort beschriebene Blutkoagulation ist bei der Behandlung von ganz oder teilweise zellvermittelten Hautentzündungen oder Akne zu vermeiden, so daß die dort beschriebene Vorrichtung für die PDT ungeeignet ist.

Aus der US 5,964,749 ist eine Bestrahlungsanordnung zur Haut-Straffung bekannt, umfassend eine Bestrahlungsquelle, die im Wellenlängenbereich von 600-1200 nm gepulstes Licht emittiert, wodurch Wärme unterhalb der Nekroseschwelle in das Gewebe eingekoppelt wird und dadurch das Collagen der Haut geschrumpft wird. Die Pulsenergien liegen dabei in der Größenordnung von 1 J/cm². Die Bestrahlungspeaks der Pulse weisen dabei eine Bestrahlungsstärke von 100-1000 W/cm² auf. Die bevorzugte Gesamtenergie, die bei einer Behandlung verabreicht wird, wird mit 100 J/cm² angegeben.

Aus der WO 00/53114 ist eine Bestrahlungsanordnung zur Haut-Straffung bekannt, umfassend eine Bestrahlungsquelle, die im Wellenlängenintervall 500-850 nm gepulstes Licht emittiert, wobei die Pulsenergie kleiner 5 J/cm² beträgt.

Aus der WO 00/28575 ist eine gattungsgemäße Bestrahlungsanordnung für therapeutische und kosmetische Zwecke zur Behandlung von primär T-Zell-vermittelten Hauterkrankungen bekannt, insbesondere von atopischer Dermatitis, cutanem T-Zell-Lymphom, Lichen ruber, Alopecia areata, systemischem Lupus erythermatodes und Psoriasis, wobei die Bestrahlungseinrichtung mindestens eine optische Strahlungsquelle umfaßt, die auf einer zu bestrahlenden Fläche im Wellenlängenintervall von 400-440 nm eine Bestrahlungsstärke von mindestens 2 mW/cm² erzeugt und im Wellenlängenintervall von 300 - 400 nm eine Bestrahlungsstärke von weniger als 21% der Bestrahlungsstärke im Wellenlängenbereich von 400- 440 nm erzeugt. Die Bestrahlungsanordnung nutzt dabei die überraschende Wirksamkeit der Strahlung im Bereich von 400 - 440 nm aus, wodurch eine Bestrahlungsanordnung zur Behandlung von primär T-Zell-vermittelten Hauterkrankungen zur Verfügung steht, mittels derer bisher kaum behandelbare Hauterkrankungen wie Lichen ruber behandelbar sind und andererseits aufgrund der um Zehnerpotenzen geringeren Karzinogenität gegenüber UVA auch eine Behandlung von Kindern ermöglicht. Weiter ist dort aufgeführt, daß für die therapeutische Wirkungsweise des blauen Lichtes patienten spezifische Schwellwerte für die Bestrahlungsstärken existieren. Als Grund hierfür wird der unterschiedliche Gehalt an Melanin und/oder Antioxidantien der Haut angegeben, so daß vorzugsweise Bestrahlungsstärken von größer 60 mW/cm² bzw. größer 100 mW/cm² zur Anwendung kommen.

Aus der EP 0 726 083 A2 ist eine therapeutische Bestrahlungsanordnung zur Behandlung von Krebszellen im Gewebe bekannt, die einen Behandlungs- und einen Diagnose-Modus aufweist. Als Strahlungsquelle wird eine breitbandige Blitzlampe verwendet, deren Spektrum je nach Modus durch Filter jeweils modifiziert wird. Im Behandlungsmodus wird im Spektralbereich zwischen 600-1000 oder 600-700 nm emittiert, wobei die Pulse eine Energiedichte zwischen 0.1-20 J/cm² aufweisen. Die Bestrahlungsstärke liegt dabei zwischen 100-2000 mW/cm². In dem Diagnose-Modus wird ausgenutzt, dass Krebszellen bei Bestrahlung mit blauem Licht fluoreszieren. Diese Fluoreszenz kann dann mit einer geeigneten Optik erfaßt und ausgewertet werden. Hierzu wird das zu untersuchende Gewebe mit Pulsen im Spektralbereich zwischen 350-500 nm, mit einem Peak bei 400 nm, bestrahlt. Die Pulsfrequenz liegt dabei zwischen 0.02-2 Hz mit einer efffektiven Pulslänge zwischen 0.1-1000 ms. Das Licht wird mit einer Energiedichte zwischen 0.02-4 J/cm² in einen Quarzstab oder einen Lichtwellenleiter eingekoppelt. Die zu diagnostizierende Fläche ist dabei abhängig von dem Abstand des Quarzstabes bzw. des Lichtwellenleiters von der Hautoberfläche. Aufgrund der Inkohärenz der Strahlung kann nur ein Bruchteil der Strahlungsenergie in den Lichtwellenleiter eingekoppelt werden. Bei dem Quarzstab kann zwar der Großteil der Energie eingekoppelt werden, jedoch weitet sich der Strahl beim Austritt extrem auf. Bei einer Fläche von beispielsweise von 0,5 cm² und einem Beobachtungsabstand von lediglich 5 cm reduziert sich die Energiedichte auf der Behandlungsfläche um den Faktor 500. Derartige Abstände sind aber notwendig, um mit der Optik die Fluoreszenz zu beobachten, was anschaulich in der US-6, 021, 344 dargestellt ist. Somit sinkt die Energiedichte auf der Haut auf 0.04-8 mJ/cm², was jedoch für die Diagnosezwecke ausreichend ist.

Der Erfindung liegt daher das technische Problem zugrunde, eine Bestrahlungsanordnung zur Behandlung von akuten und chronischen ganz oder teilweise zellvermittelten Entzündungen der Haut und der inneren Organe, viralen und anderen infektiösen Erkrankungen wie HIV oder Prionenerkrankungen, Pilzinfektionen der Haut und der Schleimhäute, bakteriellen Erkrankungen der Haut und der Schleimhäute sowie Hand- und Analekzemen zu schaffen, die gegenüber den bekannten Bestrahlungsanordnungen eine verbesserte therapeutische Wirksamkeit aufweisen.

Die Lösung des technischen Problems ergibt sich durch den Gegenstand mit den Merkmalen des Patentanspruchs 1. Weitere vorteilhafte Ausgestaltungen der Erfindung ergeben sich aus den Unteransprüchen.

Hierzu umfaßt die Bestrahlungsanordnung mindestens eine Bestrahlungsquelle zur flächenhaften Bestrahlung einer Behandlungsfläche, wobei die Wellenlänge der emittierten Strahlung auf der Behandlungsfläche größer als 400 nm ist und mindestens einen Spektralanteil im Wellenlängenintervall von 400-500 nm umfaßt und die Bestrahlungsanordnung Mittel zur Erzeugung von optischen Pulsen auf der Behandlungsfläche umfaßt, wobei die Bestrahlungsstärke pro Puls zwischen 0,5 W/cm²- 100 kW/cm² ist, wobei die Energiedichte eines emittierten optischen Pulses zwischen 0,3-0,8 J/cm² liegt. Die Angaben für die Bestrahlungsstärke und die Energiedichte beziehen sich auf die zu bestrahlende Fläche, wenn die Bestrahlungsanordnung direkt auf die Oberfläche aufgesetzt wird. Dabei wird unter flächenhaft eine Bestrahlungsfläche von größer 0,1 cm² verstanden. Die Aussage größer 400 nm bedeutet dabei, daß weniger als 7 % der optischen Gesamtleistung im UV-Bereich emittiert werden, wohingegen im Wellenlängenintervall von 400-500 nm mindestens 30 % der optischen Leistung emittiert werden. Die Anteile im UVC- und UVB-Bereich sind dabei vernachlässigbar klein im Bereich unter 0,1 % der Gesamtleistung, sodass der gesamte verbleibende UV-Anteil im UVA1- und UVA2-Bereich liegt, wobei deren Verhältnis ca. 1/10 ist, d.h. der überwiegende Anteil der verbleibenden UV-Strahlung liegt im UVA1-Bereich zwischen 340-400 nm. Weiter vorzugsweise macht der UV-Anteil weniger als 3,5-5 % der optischen Gesamtleistung und der Bereich zwischen 400-500 nm mehr als 40 % der optischen Gesamtleistung aus.

Erfindungsgemäß wird dabei ausgenutzt, daß bei einer pulsförmigen Bestrahlung in den Zeiten hoher Leistungsdichte, entgegen den Ausführungen in der Literatur, die Bildung von Singulett-Sauerstoff um Größenordnungen gegenüber cw-Betrieb bei gleicher Energie erhöht wird. Ein weiterer Vorteil der extrem erhöhten Leistungsdichte ist, daß auch tiefer unter der Haut liegende Bereiche noch eine ausreichende Bestrahlungsstärke erhalten, da üblicherweise aufgrund der geringen Eindringtiefe des blauen Lichtes nur ein Bruchteil der cw-Leistung die tieferliegenden Bereiche erreicht. Zusätzlich hat pulsförmig applizierte Strahlungsenergie eine erheblich stärkere photobiologische Wirkung. Bei gleicher integraler Gesamtdosis von cw- und Pulseinstrahlung bliebe z.B. bei einer angenommenen cw-Bestrahlungsstärke von 70 mW/cm² aufgrund der Wirkung körpereigener Antioxidatien, die z.B.60 mW/cm² der eingestrahlten Leistung als konstanten Off-Set neutralisieren, nur 10 mW/cm² für photobiologische Wirkungen verfügbar. Es ist unmittelbar einsichtig, daß die relative photobiologische Wirkungsverminderung durch diesen konstanten Off-Set durch gepulste Einstrahlung höchst effektiv vermindert werden kann. Bei Pulsleistungen im kW-Betrieb führt dieser Off-Set nicht mehr zu einer signifikanten Verminderung der Strahlungswirkung. Die mittlere Energiezufuhr wird dabei derart gewählt, daß die Nekroseschwelle für die Zellen vermieden wird und nur eine Apoptose der Zellen induziert wird. Ebenso wird die in der EP 0 565 331 B1 angestrebte Ablationsschwelle unterschritten. Eine Ablation, d.h. Gewebeverdampfung tritt immer dann auf, wenn eine Energie >2500 J/cm³ innerhalb einer Zeit im Gewebe deponiert wird, die nicht erlaubt, daß ein signifikanter Wärmeaustausch mit angrenzenden Schichten möglich ist. Aufgrund der zeitlichen Modulation durch die Pulserzeugung, wobei die Belichtungszeit unterhalb der thermischen Relaxationszeit der oberen Hautschicht liegt, kommt es zwar zu einer sehr starken Erwärmung der oberen Hautschicht, die jedoch sehr einfach abgeführt werden kann. Licht im Wellenlängenbereich 400-500 nm hat eine halbmaximale Eindringtiefe von ca. 200 µm. Die geschätzte thermische Relaxationszeit für eine Struktur mit einem Durchmesser von 200 µm beträgt ca. 20 ms. Dies bedeutet, daß bei einer Verweildauer des Lichtstrahls <20 ms lediglich die oberen Hautschichten erwärmt werden, ohne daß es zu einer Tiefendeposition der Energie kommt.

Neben den beschriebenen Anwendungsgebieten ist die Bestrahlungsanordnung auch zur Wunddesinfektion nach Verbrennungen oder zur Behandlung bei venösen Geschwüren am Unterschenkel geeignet. Diese werden bisher mit UV-Licht behandelt, wobei es häufig zu einer kurzzeitgen Verbesserung kommt. Jedoch treten häufig Komplikationen bei der langfristigen Wundheilung auf. Die Ursache für die kurzzeitigen Erfolge liegt vermutlich in der keimtötenden Wirkung von UV-Licht, wohingegen die Komplikationen aufgrund irreparabler Zellschädigungen verursacht werden. Durch eine UV-freie, zeitlich modulierte Bestrahlung duch die optischen Pulse, bevorzugt im Spektralbereich von 400-500 nm, weiter bevorzugt im Bereich von 40-480 nm und noch weiter bevorzugt im Bereich 400-420 nm kommt es in allen Zellen zwar zunächst zu einer oxidativen Schädigung, die jedoch von Eukaryontenzellen wegen der dort vorhandenen FPG-Endonukleotidasen leicht reparabel ist. Prokaryonten wie Staphylokokken oder Streptokokken sind wegen Fehlens dieser Enzyme sehr viel empfindlicher auf eine derartige Schädigung und können somit selektiv abgetötet werden. Ebenso ist die Bestrahlungsanordnung auch zur Behandlung von Akne und Aknenarben geeignet. Hierbei kommt es durch die Aktivierung körpereigener kollagenabbauender Enzyme zu einer Verflachung der Narbe. Diese Wirkungen treten auch bei der Behandlung der Sklerodermie auf, wo mit Hilfe der Bestrahlungsanordnung eine wirksame Verminderung der vernarbten Kollagen Plaques erreichbar ist. Darüber hinaus wurde bei Patienten mit Sklerodermie eine ausgeprägte Wirkung der gepulsten Bestrahlung auf die zirkulierenden Lymphozyten beobachtet. Insbesondere kam es bereits nach fünf Bestrahlungen zu einer Abnahme von Kiler-Lymphozyten von 75%. Die Anzahl der zirkulierenden Lymphozyten nahm unter der Behandlung um ca. 25 % ab. Diese Befunde sind am ehesten dadurch erklärbar, daß die entzündungsaktivierten Lymphozyten über eine Änderung ihrer endogenen Farbstoffkonzentration für die durchgeführte Bestrahlung empfindlich genug waren, um eine Apoptose auszulösen.

Die Pulse können entweder durch eine gepulste Bestrahlungsquelle und/oder durch eine Relativbewegung der Bestrahlungsquelle zu der zu behandelnden Fläche erzeugt werden.

Vorzugsweise liegen die effektiven Pulslängen zwischen 1 µs und 500 ms. Dieser relativ breite Bereich kommt daher, daß die bevorzugten effektiven Pulslängen für gepulste Bestrahlungsquellen und relativ bewegte Bestrahlungsquellen in Form eines Scanners unterschiedlich sind. Dabei ist anzumerken, daß der Scanner bevorzugt zur Behandlung größflächiger Hauterkrankungen zur Anwendung kommt.

Bei den Blitzlampen liegen die effektiven Pulslängen vorzugsweise zwischen 1 µs und 50 ms, besonders bevorzugt zwischen 10 µs und 10 ms und weiter bevorzugt zwischen 100-600 µs, wobei die Pulsein- und auszeiten unsymmetrisch sind.

Bei einer Ausbildung als Scanner liegen die effektiven Pulslängen vorzugsweise zwischen 1 ms und 500 ms, weiter vorzugsweise zwischen 20-100 ms. Unter effektiver Pulslänge wird dabei die Zeit verstanden, die zwischen Erreichen von 50 % der maximalen Leistung bis zum Abfall auf 50% der maximalen Leistung liegt. Die im Verhältnis zur effektiven Pulslänge längeren Pulsauszeiten dienen dabei insbesondere der Nachdiffusion von Sauerstoff. Das Verhältnis Pulslänge und Pulsauszeit liegt dabei vorzugsweise beim Scanner zwischen 3 und 3000 und bei der Blitzlampe zwischen 100 und 100.000. Ein weiterer Effekt ist die thermische Abkühlung der bestrahlten Fläche während der Pulsauszeiten, so daß keine Nekrose auftritt.

In einer weiteren bevorzugten Ausführungsform liegt die Frequenz, mit der die Bestrahlungsquelle gepulst wird, zwischen 0,01 - 100 Hz, weiter bevorzugt zwischen 0,05-50 Hz und noch bevorzugter zwischen 0,3-3 Hz, wobei bei höheren Frequenzen niedrigere effektive Pulslängen und kleinere Pulsenergien verwendet werden.

Um die Nachdiffusion von Sauerstoff und die thermische Abkühlung zu verbessern, wird vorzugsweise nach einer Pulsfolge von beispielsweise 100-500 Pulsen eine längere Dunkelphase von mehreren Sekunden bis Minuten eingelegt, bevor wieder erneut eine Pulsfolge erzeugt wird. Aufgrund der extrem langen Diffusionszeiten von Sauerstoff sind auch Anwendungen vorstellbar, wo nur jeweils ein Puls verabreicht wird und anschließend die Bestrahlungsanordnung wieder für eine längere Zeit abgeschaltet wird. Die Pausen können dabei im Bereich von einer bis mehreren Stunden liegen. Insbesondere zur Behandlung chronischer Erkrankungen kann dabei die Bestrahlungsanordnung beispielsweise in Form eines Gürtels, einer Bestrahlungsdecke oder eines Lichtbettes fest dem Patienten zugeordnet sein, der dann beispielsweise alle 5 Minuten einen optischen Puls erhält. Bei diesen langen Pausen sind thermische Probleme oder die Nachdiffusion von Sauerstoff vernachlässigbar.

Ebenso wie die effektiven Pulslängen davon abhängig sind, ob eine gepulste Bestrahlungsquelle oder eine relativ bewegte Bestrahlungsquelle verwendet wird, sind auch die bevorzugten Bestrahlungsstärken bzw. Leistungsdichten pro Puls unterschiedlich.

Bei Ausführungsformen mit gepulster Bestrahlungsquelle liegt die Bestrahlungsstärke pro Puls zwischen 1 W/cm²-100 kW/cm², vorzugsweise zwischen 50 W/cm²-50 kW/cm², weiter bevorzugt zwischen 500 W/cm²- 10 kw/cm² und besonders bevorzugt zwischen 1kW/cm²-5 kW/cm². Die Energiedichte pro Puls liegt dabei zwischen 50 mJ/cm²-10J/cm², vorzugsweise zwischen 100 mJ/cm²-5J/cm² und besonders bevorzugt zwischen 300 mJ/cm²-3J/cm².

Bei Ausführungsformen mit einem Scanner, wobei zusätzlich die Bestrahlungsquelle auch gepulst sein kann, liegen die Bestrahlungsstärken pro Puls zwischen 500 mW/cm²-500W/cm², vorzugsweise zwischen 1-300 W/cm² und besonders bevorzugt zwischen 50-200 W/cm².

Die über die volle Periode gemittelte cw-Bestrahlungsstärke eines optischen Pulses liegt vorzugsweise zwischen 1 mW/cm² und 10 W/cm², weiter bevorzugt zwischen 5-2000 mW/cm² und besonders bevorzugt zwischen 8-800 mW/cm². Unter gemittelter cw-Bestrahlungsstärke wird dabei der Wert verstanden, der sich ergeben würde, wenn dieBestrahlungsstärke eines Pulses gleichmäßig über die volle Periode aufgeteilt werden würde.

In einer weiteren bevorzugten Ausführungsform ist die Bestrahlungsquelle als Xe-Blitzlampe ausgebildet, der eine Einrichtung zur Unterdrückung und/oder zur Transformierung der unerwünschten Spektralanteile in den gewünschten Spektralbereich zugeordnet ist. Diese handelsüblichen Xe-Blitzlampen sind sehr preiswert und emittieren mit ausreichender Leistungsdichte im gewünschten Spektralbereich zwischen 400 - 500 nm. Hierzu wird beispielsweise auf die US 4,167, 669 oder die EP 0 565 331 verwiesen, wobei die dort beschriebenen Pulsenergien für die vorliegende Erfindung zu groß sind, da dort bewußt die Ablationsenergie überschritten wird. Die Xe-Blitzlampen sind je nach Stromdichte im Entladungskanal mehr oder weniger vom Spektrum mit einem Schwarzen Körper vergleichbar, daher emittieren diese typischerweise von 200 - 2000 nm. Dabei können die nicht gewünschten Spektralbereiche durch bekannte handelsübliche Filter herausgefiltert werden. Zur Erhöhung des erwünschten blauen Spektralbereiches werden in einer bevorzugten Ausführungsform Gallium, Indium und/oder deren Halogenide in die Xe-Blitzlampe gefüllt. Des weiteren kann die Xe-Blitzlampe auch mit Qucksilber, Quecksilberjodid oder Amalgam dotiert werden, um den Wirkungsgrad im blauen Bereich zu erhöhen. Bei reinen Xe-Blitzlampen wurden besonders gute Ausbeuten mit ca. 40 mm Elektrodenlängen, bei einem Durchmesser von 3,2 mm erreicht, wobei die Betriebsspannung bei ca.600 V lag. Alternativ zur Xe-Blitzlampe können auch Deuterium-Blitzlampen Anwendung finden.

Eine weitere mögliche Bestrahlungsquelle ist eine im Überlastbereich gepulste galliumjodid-Quecksilberentladungslampe. Dabei wird unter Überlast verstanden, wenn der maximale Entladungsstrom mindestens 3-1000 fach über dem Lampennennstrom liegt, wobei der Impulsentladungsstrom vorzugsweise zwischen 15-1500 A/cm² Querschnittsfläche des Entladungsgefäßes liegt. Handelsübliche cw-betriebene metalldampfdotierte Quecksilberhalogenid-Lampen sind beispielsweise in der US-3,521,111; US-3,540,789 und der WO 96/13851 beschrieben.

Aus der US 5,184,044 ist zu entnehmen, daß unter Berücksichtigung der Lampengeometrie, der Lampenleistung von 20 W und dem Spannungsabfall von 55 V ein Lampenstrom von 8 A/cm² Querschnittsfläche des Entladungsgefäßes der maximal sinnvollen Lampenbelastung entspricht, da es bereits zu einer Inversion im Bereich der Indiumresonanz kommt. Eine weitere Erhöhung der Stromdichte würde die Inversion bis zur Löschung verstärken.

Deshalb wurden diese bisher nicht im Überlastbetrieb gefahren, da es aus plasmaphysikalischen Untersuchungen bekannt war, dass schmalbandige Emissionsspektren nur bei vergleichsweise niedriger Anregungsenergie darstellbar sind. Im Überlastbetrieb steigt der Dampfdruck im Entladungslasma so stark an, dass die Linienemission vom Umgebungsplasma absorbiert wird und es paradoxerweise zu einer erheblichen Schwächung oder sogar einer kompletten Löschung der Resonanzlinienemission bei hohen Entladungsdrücken kommt. Als Beispiele seien die Quecksilberresonanzlinie bei 254 nm, die Natriumresonanzlinie bei 488 nm und die Indiumresonanzlinie bei 450 nm genannt, wo es schon bei mäßiger Überlast zu einem Verlust der Spektralintensität kommt.

Überraschend wurde jedoch entdeckt, daß galliumdotierte Quecksilberjodid-Mittel- bzw. Hochdrucklampen selbst bei einer 100-1000 fachen Überlast weder eine Verbreiterung oder gar Inversion der Galliumresonanzlinien bei 403 und 417 nm zeigen. Eine unter Nennlast mit einem Entladungsstrom von 1,5 A/cm² Querschnittsfläche des Entladungsgefäßes betriebene galliumjodiddotierte Quecksilberentladungslampe konnte im Pulsbetrieb bis auf 1000 A/cm² Querschnittsfläche des Entladungsgefäßes betrieben werden, ohne daß es zu einer Abschwächung oder Frequenzinversion der Galliumresonanzlinien kam. Eine mögliche Erklärung hierfür ist, daß das metallische Gallium einen Siedepunkt von ca. 2200°C aufweist, so daß der diesbezügliche Galliumdampfdruck auch unter Impulsbedingungen vermutlich zu vernachlässigen ist. Im Plasma kommt es zu einer Zersetzung des Quecksilberiodids in Quecksilber und Jod. Das Jod verbindet sich unter den Entladungsbedingungen mit dem Gallium in Form der instabilen Verbindung Gal₃. Galliumiodid zeigt eine starke Dampfdruckzunahme bereits bei niedrigen Temperaturen. Die fehlende Inversion der Galliumresonanzlinie wäre nun dadurch erklärbar, dass das Galliumiodid sich nur bis zu einem bestimmten Druck stabil verhält und es bei einer Druckzunahme zu einem massiven, vermutlich kinetisch außerordentlich schnellem Zerfall der Verbindung kommt. In der Folge stellt sich trotz zunehmender hoher Temperaturen während des Impulses ein relativ konstanter Galliumdampfdruck ein. Somit trägt das kondensierte Gallium nicht zur Entladung bzw. zur möglichen Selbstabsorption bei. Der unerwartet aufgetretene Effekt könnte deshalb mit einer paradoxen Konstanz des Galliumdampfdrucks über einen Temperaturbereich von 200 bis fast 2200° C zusammenhängen. Quecksilberjodid zerfällt bereits früh in Quecksilber und Jod, so dass das Jod für eine Galliumverbindung zur Verfügung steht und der Quecksilberdampfdruck mit der eingespeisten Energie stark zunimmt. Auf diese Weise steht der Galliumresonanz entsprechend dem Wirkungsquerschnitt des entsprechenden Galliumplasmas eine proportional zur eingespeisten Energie zunehmende Anregung durch die kurzwelligen Quecksilberelektronenübergänge zur Verfügung. Diese Energie kann wegen der relativen Konstanz des Galliumdampfdruckes fast in der gesamten Höhe als Resonanzlinienspektrum emittiert werden.

Während des Überlastbetriebes kommt es zu einer kurzzeitigen Überhitzung, insbesondere der Wolframwendeln, die jedoch bei einer Zunahme der Temperatur entsprechend dem Planckschen Gesetz erheblich mehr Wärme abstrahlen können. Daher kann eine modulierte Lampe mit einer erhöhten Grundlast betrieben werden, da die Abgabe der zugeführten Energie nur aufgrund der Temperaturerhöhung erheblich effizienter ist als bei einer Lampe im Normalbetrieb. Dabei hat sich herausgestellt, dass sich eine 1 kW Lampe mit einer Dauerlast von ca. 2-20 kW betreiben läßt. Bei spektralen Messungen hat sich gezeigt, daß bei einer 1000 W cw-Betrieb galliumdotierten Quecksilberiodid-Lampe ca. 400 mW/m² im Spektralbereich zwischen 400-440 nm die Haut treffen. Diese Bestrahlungsstärke läßt sich im Simmerbetrieb auf eine gemittelte Bestrahlungsstärke von ca.2-4 mW/cm² vermindern, wobei unter Impulslast die Bestrahlungsstärke kurzzeitig um bis zu vier bis fünf Größenordnungen angehoben wird, so daß Bestrahlungsstärken zwischen 2 bis 400 W/cm² auf die Haut treffen. Das Verhältnis der Pulslängen liegt vorzugsweise im Bereich zwischen 3 und 300. Diese einfache Impulslichtquelle im Spektralbereich von 320-540 nm ist auch für andere technische Anwendungen verwendbar, wie beispielsweise drucktechnische Anwendungen, Oberflächenversiegelungen, Rohrsanierung mit lichthärtenden Schläuchen, Kunststoffhärtung im Bereich der DVD-Produktion sowie die Beschleunigung anderer photochemischer Reaktionen, die über Radikalmechanismen einer Photoabsorption im UV-Blaubereich des Spektrums beeinflußt werden.

Das Verhältnis Gallium bzw. Galliumadditiv zu Quecksilber sollte vorzugsweise zwischen 1:10 bis 1:100 sein. Im Leistungsbereich von 400 W kommt vorzugsweise ein Mischungsverhältnis von 1-5 mg Galliumiodid auf 44 mg Quecksilber zur Anwendung.

Eine andere typische Lampe besteht aus einem zylindrischen Quarzrohr mit Durchmesser 13,5 mm und einem Volumen des Entladungsgefäßes von 20 cm³. Der Elektrodenabstand beträgt ca.14 cm. Eine derartige Lampe wird mit 20 mg Hg, 3 mg Quecksilberiodid, 1 mg Gallium und Argon mit einem Druck von 3,57 mmHg befüllt.

Die UV-Anteile können jedoch auch in den gewünschten Spektralbereich transformiert werden. Dabei haben sich besonders aus Silikonelastomeren oder Fluorpolymeren, insbesondere Teflon bestehende Folien mit anorganischen Leuchtstoffen bewährt. Die Silikonelastomere werden vorzugsweise durch eine Additionsvernetzung hergestellt, sodass keine flüchtigen Bestandteile wie beispielsweise Wasser mit den anorganischen Leuchtstoffen in Berührung kommen. Das Silikonelastomer wird vorzugsweise durch eine Mischung eines Hydroxylpolydiorganosiloxans mit einem Organohydrogensiloxans hergestellt, wobei die Leuchtstoffe zugemischt werden und anschließend eine chemische Reaktion mittels eines Platinkatalysators bei Zimmertemperatur erzeugt wird. Die Leuchtstofffolie weist dabei vorzugsweise eine Dicke von 10-800 µm auf, wobei die Flächendichte der Leuchtstoffpartikel vorzugsweise zwischen 1-20 mg/cm² bei einer Korngröße von 5-15 µm liegt. Prinzipiell kann der UVC-transparente Träger auch als Silikonkautschuk ausgebildet sein, der ohne Wärme- und Druckzufuhr aushärtet. Aufgrund der nicht unerheblichen Wärmeeinfuhr in das Trägermaterial hat es sich insbesondere bei den Silikonelastomeren als vorteilhaft erwiesen, diese zu kühlen, was deren Lebensdauer um Größenordnungen verlängert. Je nach Wärmeeinfuhr kann die Kühleinrichtung als Luftgebläse oder als Wasserbad ausgebildet sein, in der die Folie liegt.

In einer weiteren bevorzugten Ausführungsform wird die gepulste Bestrahlungsquelle in einem Simmer betrieben, wodurch sich die erreichbare Flankensteilheit der Pulse erhöhen läßt.

Alternativ kann die Erzeugung von Pulsen der emittierten Strahlung durch eine Einrichtung realisiert werden, mittels derer die zu bestrahlende Fläche relativ zur Bestrahlungsquelle bewegbar ist. Im einfachsten Fall kommt hierbei ein X-oder X-Y-Scantisch zur Anwendung, mittels dessen der Patient unter der im cw-Betrieb betreibbaren Bestrahlungsquelle hin- und herbewegt wird. Als Bestrahlungsquelle für den cw-Betrieb kommen prinzipiell alle vorzugsweise im blauen Bereich emittierenden Bestrahlungsquellen wie beispieleweise blauemittierende LEDs oder entsprechende Gasentladungslampen, die vorzugsweise Gallium, Indium oder deren Halogenide beinhalten in Frage. Bei der Scan-Geschwindigkeit ist ebenfalls darauf zu achten, daß die Ablationsschwelle ebenfalls nicht überschritten wird. Hierzu wird beispielsweise eine Brennlinie einer Dicke mehrerer Millimeter erzeugt, die dann mit einer Geschwindigkeit von 1-100 cm/s quer oder längs über die Behandlungsfläche verfahren wird.

Es ist jedoch auch möglich, die Scanbewegung mit einer gepulsten Bestrahlungsquelle zu kombinieren, so daß die erreichbaren effektiven Pulslängen für eine zu bestrahlende Fläche weiter verkleinert werden können, was insbesondere im Hinblick auf die thermische Relaxation vorteilhaft ist.

Insbesondere im oberen Leistungsbereich ist die Bestrahlungsanordnung mit einer Einrichtung zur Kühlung der zu bestrahlenden Fläche ausgebildet, um eine Nekrose der bestrahlten Zellen zu verhindern. Dabei muß eine Erwärmung der Zellen über 60 °C verhindert werden. Art und Umfang der Kühlung sind dabei abhängig, welche Energien in welcher zeitlichen Abfolge verabreicht werden. Im Regelfall ist eine einfache Luftkühlung mit einem gegebenenfalls gekühlten Luftstrom völlig ausreichend. Bei sehr hohen Energien kann die Luftkühlung durch eine Kontaktkühlung ersetzt werden, die beispielsweise durch einen gekühlten Saphir oder direkt auf die Haut aufgesprühte Kühlmittel erzeugt wird. Andere Möglichkeiten für eine Kontaktkühlung ist die Verwendung gekühlter Flüssigkeiten wie beispielsweise Wasser, Öle oder Alkohol, die über eine Membran aus Latex oder Silikon die Wärme dem Gewebe entziehen. Die Kühlmittel müssen dabei optisch transparent sein, wobei der Wärmeübergangswiderstand möglichst klein ist. Je stärker die Haut abgekühlt werden kann, ohne daß es zu Gefrierungsschäden kommt, desto mehr Energie kann eingestrahlt werden, ohne die Nekroseschwelle der Zellen zu überschreiten. Dabei kommt ein weiterer Vorteil der Pulse zum Tragen. Die Gradienten der Wärmeeinfuhr durch die optischen Pulse und der Kältezufuhr durch die Kühlung sind im Gewebe unterschiedlich. Dabei ist der Gradient der Kältezufuhr im Regelfall flacher, so daß es zu einem Gefrierschaden aufgrund einer Kristallisationsbildung kommen könnte. Durch die Pulse kommt es jedoch zu einer Schockerwärmung im Bereich von 100 µs, so daß die Eisbildung trotz der tiefen Temperaturen gestört wird. Vorzugsweise erfolgt die Kühlung synchron zur Wärmeeinfuhr, d.h. die Kälteleistung wird während des Pulses erhöht. Diese Steuerung kann beispielsweise mittels eines Peltierelementes erfolgen, wobei die Temperatur des Kühlmittels von beispielsweise 4°C während des Pulses auf -(40-80)°C erniedrigt wird.

Die Effizienz der Bestrahlungsanordnung kann weiter durch eine Erhöhung der Sauerstoffkonzentration erhöht werden. Neben den in der WO 00/02491 beschriebenen Maßnahmen kann dies auch sehr einfach durch eine inspiratorische Sauerstoffzufuhr über eine Sauerstoffmaske erfolgen. Der Vorteil der inspiratorischen Sauerstoffzufuhr ist, daß auch tieferliegende Gewebebereiche durch die Blutzirkulation verstärkt mit Sauerstoff versorgt werden, wohingegen bei der topischen Sauerstoffzufuhr stets ein Gradient von der Hautoberfläche zu den Zellen zu berücksichtigen ist.

Die mittlere Eindringtiefe des Lichtes ist stark abhängig von der Wellenlänge, wobei die Eindringtiefe mit der Wellenlänge zunimmt. Daher wird in einer bevorzugten Ausführungsform zusätzlich im Bereich von 520 - 550 nm und/oder 610 - 670 nm emittiert, was beispielsweise sehr leicht durch Beigabe entsprechender Leuchtstoffe in die Leuchtstoffolie realisierbar ist. Dabei wird der Rot- bzw. Gelb-Anteil zur Bestrahlung tieferliegender Zellen auf Kosten des Blau-Anteils erhöht. Der Blau-Anteil hat jedoch auch bei tieferliegenden Entzündungen einen wichtigen Anteil, da dieser oberflächlich befindliche Bakterien abtötet, die häufig als Folge der Entzündung sich ansiedeln und sogenannte Superantigene produzieren, die selbst wieder entzündungsfördernd sind.

Zur Erhöhung der abgestrahlten Leistung in Richtung der zu behandelnden Fläche wird die Strahlungsquelle vorzugsweise mit einem Reflektor ausgebildet. Dieser kann beispielsweise als Paraboloid- oder Ellipsoid-Reflektor ausgebildet sein. Der Paraboloid-Reflektor findet vorzugsweise Anwendung, wenn die zeitliche Modulation der emittierten Strahlung durch Pulsbetrieb realisiert wird, wohingegen der Ellipsoid-Reflektor vorzugsweise beim Scan-Betrieb verwendet wird.

Vorzugsweise ist der Strahldurchmesser der emittierten Strahlung größer 4 mm, vorzugsweise größer als 10 mm und besonders bevorzugt größer als 40 mm. Dabei wird ausgenutzt, daß die Eindringtiefe des Lichtes auch von der Größe der zu bestrahlenden Fläche abhängig ist. Insbesondere bei nahezu punktförmiger Bestrahlung ist die Eindringtiefe nur sehr gering ist. Bei flächenhafter Bestrahlung kommt es trotz der Streuung in den oberen Hautschichten zu einer additiven Überlagerung benachbarter gestreuter Photonen. In der Folge ist die resultierende Eindringtiefe wesentlich größer als bei punktförmiger Einstrahlung bei gleicher Flächenteistung. Allerdings sollte der Strahldurchmesser auch nicht zu groß gewählt werden und sollte daher 200 mm, bevorzugt 100 und besonders bevorzugt 60 mm nicht überschreiten. Dem liegen folgende Überlegungen zugrunde.

Durch Aufweitung des Bestrahlungsfeldes sinkt die Flächenleistung pro Zeit, so daß die Verweildauer des aufgeweiteten Bestrahlungsfeldes größer sein kann. Hierdurch wird erreicht, daß eine wesentlich größere Anzahl absorbierender Chromophore über einen wesentlich größeren Zeitraum als bei einem Kurzpuls photochemisch angeregt werden kann. Das Fehlen von Bestrahlungspeaks innerhalb der Bestrahlungsfläche verhindert das lokale Ausbleichen bzw. den lokalen Substratmangel von Sauerstoff. Darüber hinaus kommt es zur Ausbildung eines lokalen Maximums im Zentralbereich des Bestrahlungsfeldes, da die ungerichtete Streuung sämtlicher Strahlen zu einer Aufaddition im zentralen Bereich führt. In Abhängigkeit von den Gewebeparametem und dem Spektrum hat das optimale Bestrahlungsfeld einen Durchmesser >4 mm und < 60 mm, da bei diesem großen Durchmesser die Streuung der Randstrahlen nicht mehr zur Erhöhung der Intensität im zentralen Bereich beiträgt. Durch Wahl eines optimalen Strahldurchmessers wird somit eine höhere Intensität im zentralen Bereich bzw. eine größere Eindringtiefe ermöglicht. Bei weiterer Ausdehnung des Bestrahlungsfeldes sinkt die Flächenleistung proportional zur Zunahme der Fläche ab, so daß in den tieferen Gewebebereichen keine Lichtwirkung gemessen werden kann. Darüber hinaus ist die Zeit bis zur Wiederbestrahlung der immer größer werdenden Gewebeflächen immer kürzer, so daß die Wärmeabfuhr bzw. die Kühlung erschwert wird.

Die Erfindung wird nachfolgend anhand eines bevorzugten Ausführungsbeispiels näher erläutert. Die Fig. zeigen:

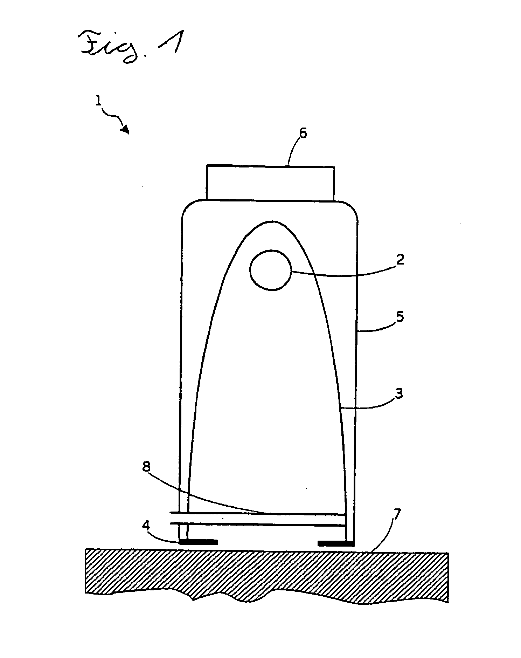

- Fig.1: einen Querschnitt durch eine Bestrahlungsanordnung mit einer pulsbaren Bestrahlungsquelle,
- Fig.2: ein Spektrum der Bestrahlungsquelle mit Leuchtstofffolie,
- Fig.3: eine schematische Darstellung einer Bestrahlungsanordnung mit einer Winkelbewegung der Bestrahlungsquelle,
- Fig.4: eine schematische Darstellung einer Bestrahlungsanordnung mit einer eindimensionalen Scan-Bewegung,
- Fig.5: eine schematische Darstellung einer Bestrahlungsanordnung mit einer zweidimensionalen Scan-Bewegung,
- Fig.6a-c: verschiedene Darstellungen der Eindringtiefe über der Bestrahlungsfläche,
- Fig.7: eine Darstellung der Eindringtiefe über der Wellenlänge,
- Fig.8a-c: verschiedene Bestrahlungsfolgen,
- Fig.9: Verläufe der relativen Bestrahlungsstärke einer galliumdotierten Quecksilberiodid-Lampe in cw-Betrieb und gepulstem Überlastbetrieb,
- Fig.10: die spektrale Energiedichte einer galliumdotierten Quecksilberiodid-Lampe bei verschiedenen Eingangsleistungen,
- Fig.11: Verläufe der relativen Bestrahlungsstärke einer Natriumdampflampe im cw- Puls-Überlastbereich,
- Fig.12: eine schematische Schaltungsanordnung zum Impulsbetrieb einer galliumdotierten Quecksilberiodid-Lampe mit zwei Phasen eines Drehstromnetzes und
- Fig.13: eine alternative Schaltungsanordnung mit einer Kondensatorbank.

Die Bestrahlungsanordnung 1 umfaßt eine pulsbare Bestrahlungsquelle 2, die vorzugsweise als Xe-Blitzlampe ausgebildet ist. Die Bestrahlungsquelle 2 ist in einem Brennpunkt eines Paraboloid-Reflektors 3 angeordnet, der an der dem Brennpunkt abgewandten Seite offen ist. Die Austrittsfläche am offenen Ende des Paraboloidreflektors 3 wird durch eine vorzugsweise verstellbare Blende 4 definiert. Durch die verstellbare Blende 4 kann somit die Größe der zu bestrahlenden Fläche angepasst werden. Die Bestrahlungsquelle 2 und der Paraboloid-Reflektor 3 sind in einem Gehäuse 5 angeordnet. Das Gehäuse 5 ist vorzugsweise mit einem Handstück 6 ausgebildet, mittels dessen die Bestrahlungsanordnung 1 einfach auf eine zu behandelnde Fläche 7 aufsetzbar ist. Zwischen der Bestrahlungsquelle 2 und der zu behandelnden Fläche 7 ist eine Leuchtstofffolie 8 angeordnet, die mit Leuchtstoffpartikeln dotiert ist. Die Leuchtsstofffolie 8 kann auch unmittelbar im Bereich der Bestrahlungsquelle 2 oder aber über die Blende 4 gespannt sein. Vorzugsweise ist die Leuchtstofffolie 8 derart angeordnet, daß diese leicht auswechselbar ist. Dies vereinfacht den notwendigen Austausch aufgrund von Alterungsprozessen, aber auch den flexiblen Einsatz von Leuchtstofffolien mit unterschiedlichen Leuchtstoffpartikeln. Des weiteren kann bei äußerer Anordnung der Leuchtsstofffolie 8 diese leicht desinfiziert werden. Die elektrischen Anschlüsse und eine Schaltung zur Erzeugung einer variablen Pulsbreite ist hier aus Übersichtsgründen nicht dargestellt.

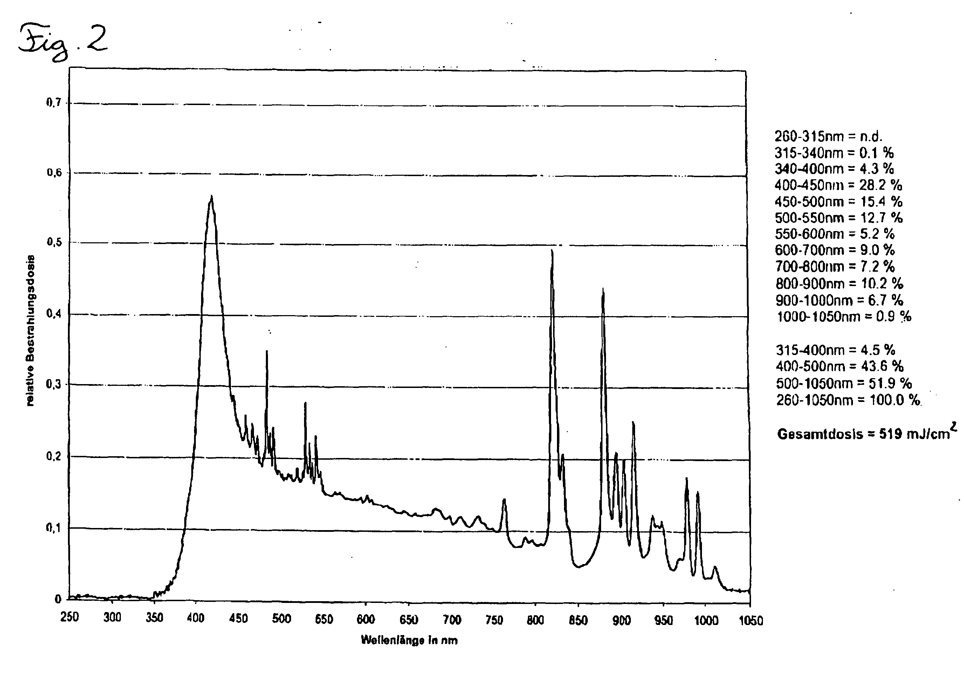

In der Fig.2 ist ein Spektrum einer verwendeten Xe-Blitzlampe mit Leuchtsstofffolie dargestellt, wobei das Entladungsrohr aus Quarzglas besteht. Bei der Leuchtstofffolie handelt es sich um ein Silikonelastomer, das mit anorganischen Leuchtstoffen dotiert ist, die im blauen Spektralbereich von 400-450 nm bevorzugt emittieren. Die Leuchtsstofffolie schneidet dabei den UV-Bereich zwischen 280-400 nm nahezu ab und transformiert diesen in den sichtbaren blauen Bereich von 400-450 nm. Im dargestellten Beispiel ist die Energie im Bereich von unter 400 nm kleiner als 4,5 % der optischen Gesamtleistung. Darüber hinaus liegt die UV-Strahlung nahezu ausschließlich im Bereich von 340-400 nm und dabei insbesondere im Bereich zwischen 370-400 nm. Da dieser Wellenlängenbereich eine um Größenordnungen geringere photobiologische Wirksamkeit aufweist als der UVB- oder UVC-Bereich, werden die internationalen Grenzwerte für UV-Belastungen nicht überschritten. Hinsichtlich der Definition sei beispielsweise auf ICNIRP (IRPA) - International Commission on Non-Ionizing Radiation Protection Association "Guidelines on limits of exposure to ultraviolet radiation of wavelengths between 180 nm and 400 nm", Health Physics 49: 331-340, 1985 oder "Proposed change to the IPRA 1985 guidelines on limits of exposure to ultraviolet radiation" Health Physics 56:971-972, 1989 verwiesen. Die optische Energie im Wellenlängenintervall zwischen 400-500 nm beträgt 43,6 % und im Wellenlängenintervall zwischen 400-450 nm 28,2 % der optischen Gesamtleistung. Die Messung wurde dabei mit einem kalibrierten CDI-Spektrometer und 100 µm-UV-Faser durchgeführt.

Die Xe-Blitzlampe wird mit einer Frequenz von 0,01-100 Hz getaktet, wobei jedoch die effektiven Pulslängen zwischen 10 µs und 1 ms liegen. Die Energie der einzelnen Pulse liegt dabei im Bereich von 0,3-0,8 J/cm².

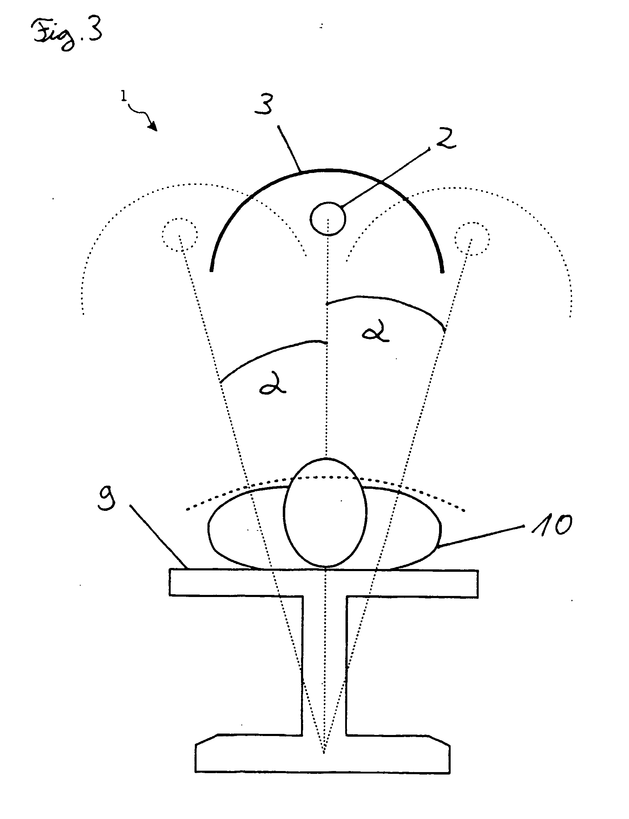

In der Fig.3 ist eine alternative Ausführungsform der Bestrahlungsanordnung 1 zur Erzeugung einer zeitlichen Lichtmodulation dargestellt. Die Bestrahlungsanordnung 1 umfaßt eine Patientenruheeinrichtung 9, oberhalb derer die Bestrahlungsquelle 2 angeordnet ist. Die Bestrahlungsquelle 2 ist wieder von einem Paraboloid-, Ellipsoid- oder einem Halbzylinderreflektor 3 umgeben. Die Bestrahlungsquelle 2 ist zusammen mit dem Reflektor 3 mittels einer nicht dargestellten Schwenkvorrichtung um den Winkel α aus der vertikalen Position zur Patientenruheeinrichtung 9 nach links und nachts verschwenkbar. Durch diese Schwenkbewegung werden nacheinander unterschiedliche Körperbereiche eines auf der Patientenruheeinrichtung liegenden Patienten 10 bestrahlt, so daß für einen einzelnen Körperbereich eine zeitliche Lichtmodulation realisiert wird. Die Bestrahlungsquelle kann dabei wieder gepulst werden oder aber auch im cw-Betrieb betrieben werden. Alternativ oder kumulativ zur Schwenkbewegung der Bestrahlungsquelle 2 kann auch die Patientenruheeinrichtung 9 geschwenkt werden.

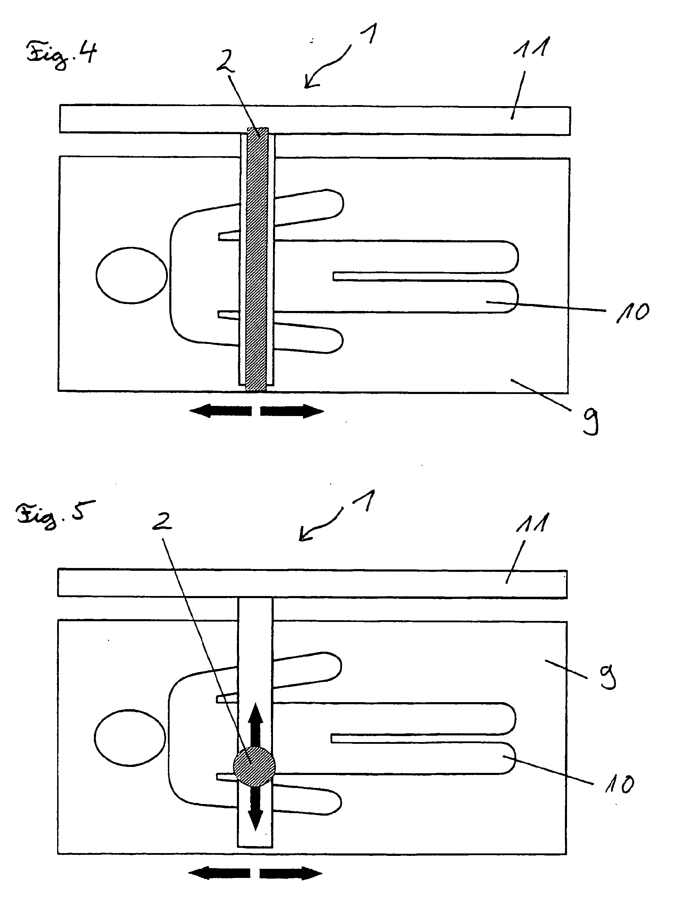

In der Fig.4 ist eine weitere alternative Ausführungsform der Bestrahlungsanordnung 1 dargestellt. Die Bestrahlungsquelle 2 ist hierbei linien- bzw. streifenförmige Bestrahlungsquelle 2 ausgebildet und oberhalb der Patientenruheeinrichtung 9 an einem Träger 11 in Pfeilrichtung verfahrbar angeordnet. Die Bestrahlungsquelle 2 kann dabei im Puls- oder cw-Betrieb betrieben werden. Durch diese Scan-Bewegung in Pfeilrichtung wird ebenfalls eine zeitliche Lichtmodulation für jede einzelne Körperpartie erreicht. Wie in Fig.5 dargestellt, kann die eindimensionale Scan-Bewegung aus Fig.4 auch durch eine zweidimensionale Scan-Bewegung ersetzt werden. Hierzu ist die flächenhafte Bestrahlungsquelle 2 noch zusätzlich quer zum Patienten 10 verfahrbar.

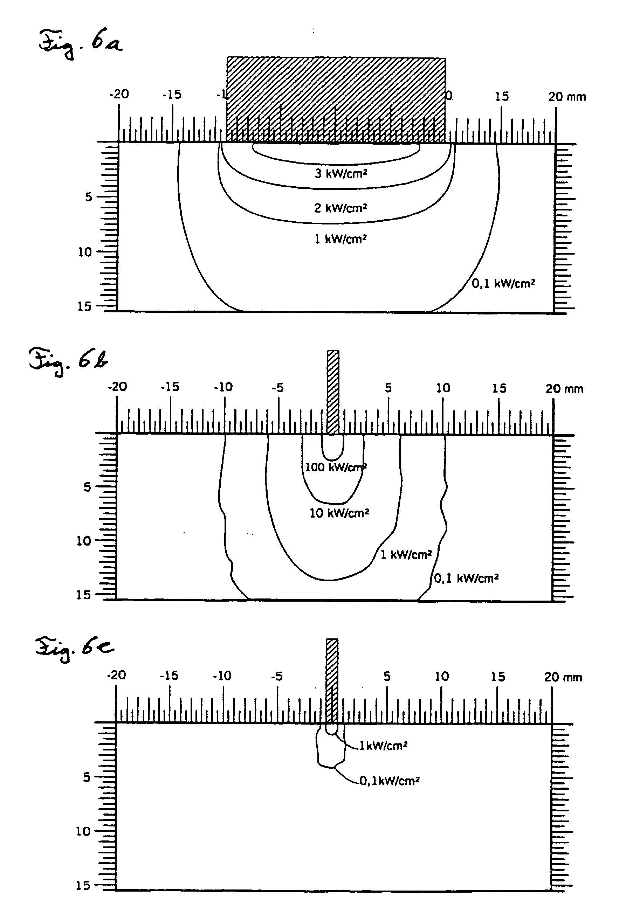

Die Notwendigkeit einer flächenhaften Bestrahlungsquelle soll anhand der Fig.6a-c näher erläutert werden. In der Fig.6a ist im Querschnitt dargestellt, welche Leistungsdichten in welcher Eindringtiefe anzutreffen sind, wenn die Lichtleistung mit einem Strahldurchmesser von 20 mm eingestrahlt wird. Wie ersichtlich ist die Leistungsdichte in 15 mm Tiefe noch 0,1 kW/cm². In Fig.6b sind die Verhältnisse dargestellt, wenn die gleich Gesamtleistung über einen Strahlendurchmesser von 1 mm in das Gewebe eingekoppelt wird. Wird eine quadratische Bestrahlungsfläche angenommen, so hat sich die Leistungsdichte entsprechend vervierhundertfacht. Dies führt jedoch zu einem riesigen Gradienten der eindringenden Leistungsdichte, wobei insbesondere unmittelbar an der Gewebeoberfläche die Leistungsdichte mit 100 kW/cm² so hoch ist, daß es zu einer Ablation kommt. Wird hingegen über den Strahlendurchmesser von 1 mm die gleiche Flächenleistungsdichte wie in Fig.6a eingekoppelt, so gelangt kaum noch optische Leistung in tieferliegende Geweberegionen, was in Fig.6c dargestellt ist. Bereits nach 5 mm Eindringtiefe ist die Leistungsdichte auf 0,1 kW/cm² abgefallen. Anhand der Fig.6b und 6c wird deutlich, daß bei kleinen Strahlendurchmessem keine große Eindringtiefen erreichbar sind, ohne an der Oberfläche Ablation zu vermeiden.

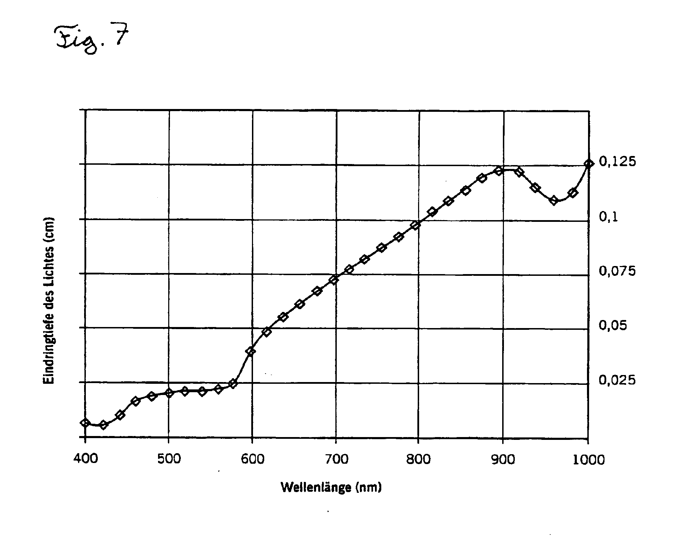

In der Fig.7 ist spektrale Abhängigkeit der Eindringtiefe (1/e-Abfall) dargestellt. Wie ersichtlich steigt die Eindringtiefe von 400 nm bis zu 900 nm stetig, so daß insbesondere bei der Behandlung von tieferliegenden Zellen es vorteilhaft ist, den grünen und roten Bereich des emittierten Spektrums auf Kosten des blauen Anteils zu erhöhen, auch wenn die Absorption der Porphyrine gegenüber dem blauen Anteil schlechter ist.

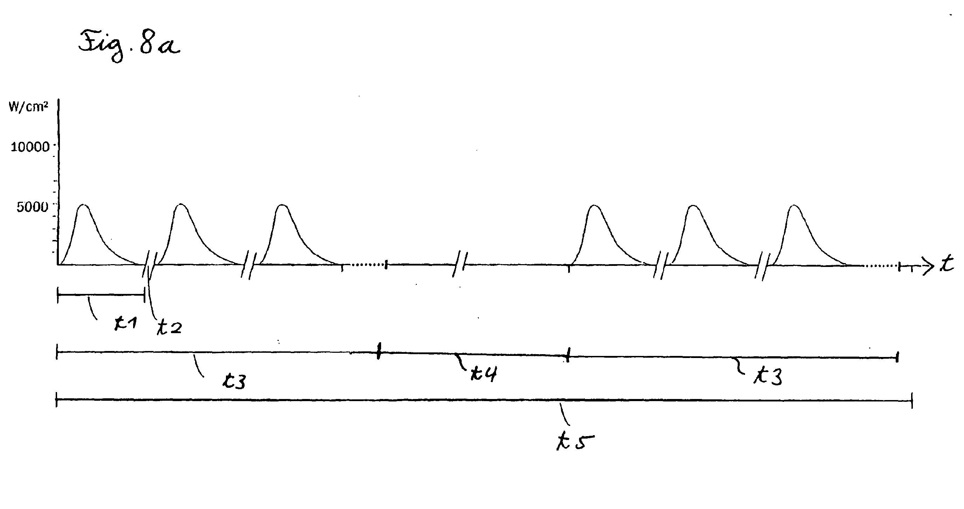

In der Fig.8a ist eine bevorzugte Ausführungsform einer Bestrahlungstherapie mit einer pulsbaren Bestrahlungsquelle mit einem Bestrahlungspeak von 5 kW/cm² dargestellt. Dabei wird eine Folge von Pulsen bzw. Blitzen emittiert.

Der einzelne Puls weist dabei eine effektive Pulslänge t1 zwischen 100-2000 µs auf, an die sich eine Pulsauszeit t2 zwischen 10ms-1000s anschließt. Die effektive Pulslänge t1 liegt dabei vorzugsweise zwischen 100-500 µs und die Pulsauszeit t2 zwischen 100 ms- 4 s. Die Anzahl der Pulse liegt dabei vorzugweise zwischen 10 und 10000, besonders bevorzugt zwischen 100 und 1000. Die Gesamtpulsfolgezeit t3 ergibt sich entsprechend (t1+t2) multipliziert mit der Anzahl der Pulse. An diese erste Pulsfolge schließt sich eine Zeit t4 an, in der nicht bestrahlt wird, so daß Sauerstoff nachdiffundieren kann und sich das Gewebe gleichzeitig abkühlen kann, was die Nekrosebildung verhindert. Die Zeit t4 wird vorzugsweise zwischen 1 min und 100 min gewählt, wobei die größeren Zeiten besonders bevorzugt sind. Anschließend wird erneut eine Pulsfolge mit der Pulsfolgezeit t3 erzeugt. Daran schließt sich wieder eine Zeit t4 an. Die Gesamtbestrahlungszeit t5 wird dabei je nach Schwere der Erkrankung zwischen einigen Minuten und 2 Stunden gewählt. Nach einer längeren Pause von mehreren Stunden bzw. 1-3 Tagen wird dann der gesamte Vorgang wiederholt. Auf die innere oder äußere Zugabe von zusätzlichen Farbstoffmolekülen wird dabei gänzlich verzichtet, sodass das beschriebene Verfahren keine PDT ist.

Die Verhältnisse sollen an einem noch konkretem Beispiel zur Behandlung von allergischen Kontaktekzemen näher erläutert werden. Hierbei werden zwei Bestrahlungszyklen pro Tag durch geführt, wobei t3 und t4 jeweils zu 5 Minuten gewählt sind, so daß sich eine Gesamtbehandlungszeit von 15 Minuten pro Tag ergibt. Die Pulsfrequenz beträgt 0,5 Hz, so daß in einem Zeitraum t3 150 Pulse appliziert werden. Die effektive Pulslänge t1 beträgt 100 µs bei einer Anstiegszeit von ca. 10 µs. Somit liegt die Pulsauszeit t2 bei ca. 2s. Der Bestrahlungspeak der Pulse liegt bei 0,5 kW/cm², wobei die Energiedichte pro Puls je nach Flankensteilheit zwischen 0,4-0,5 J/cm² liegt. Damit ergibt sich eine gemittelte cw-Leistung von 250 mW/cm². Somit beträgt die pro Tag applizierte Energiedichte 120-150 J/cm². Bei insgesamt 2 Bestrahlungen wöchentlich ist die Gesamtenergiedichte 240-300 J/cm², wobei die Behandlung vorzugsweise 4-8 Wochen dauert.

Die beschriebene Bestrahlungstherapie wurde darüber hinaus mit einer Frequenz von 0,05 Hz durchgeführt, wobei die Werte für t1, t3 und t4 sowie des Bestrahlungspeaks beibehalten wurden. Aufgrund der Verzehnfachung von t2 erniedrigen sich die applizierte Energiedichte pro Behandlungszyklus und die gemittelte cw-Leistung um den Faktor zehn, wobei die Behandlungsergebnisse-Ergebnisse vergleichbar waren. Dies liegt vermutlich an der sehr langsamen Sauerdiffusion, so daß ohne zusätzliche Sauerstoffzufuhr eine beliebige Erhöhung der Energiedichte in gleichen Zeiten kaum noch einen zusätzlichen therapeutischen Effekt bewirkt.

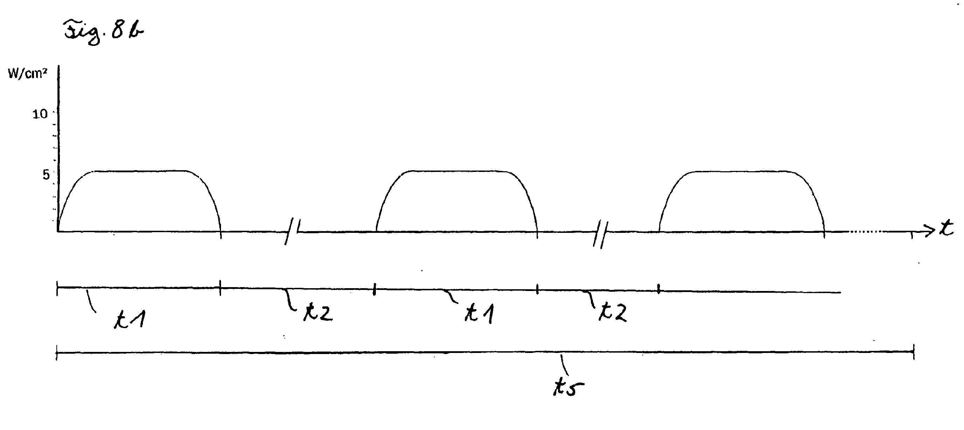

In der Fig.8b ist der Bestrahlungszyklus mit einer Bestrahlungsanordnung gemäß Fig.4 dargestellt, wobei die Bestrahlungsquelle im cw-Betrieb betrieben wird. Der Bestrahlungspeak liegt dabei bei 5 W/cm², also erheblich niedriger als beim Pulsbetrieb gemäß Fig.8a. Die Zeit t1 entspricht dabei der Zeit, die die Bestrahlungsquelle aufgrund ihrer flächenhaften Bestrahlung ein bestimmtes Gebiet während des Scan-Vorganges bestrahlt und liegt vorzugsweise zwischen 0,1 und 0,5 s. Die Zeit t2 ist die Zeit eines kompletten Scan-Vorganges abzüglich der Zeit t1. Während dieser Zeit kann wieder Sauerstoff nachdiffundieren und sich das Gewebe abkühlen. t2 liegt zwischen 1 und 300 Sekunden, vorzugsweise zwischen 2-20 Sekunden.

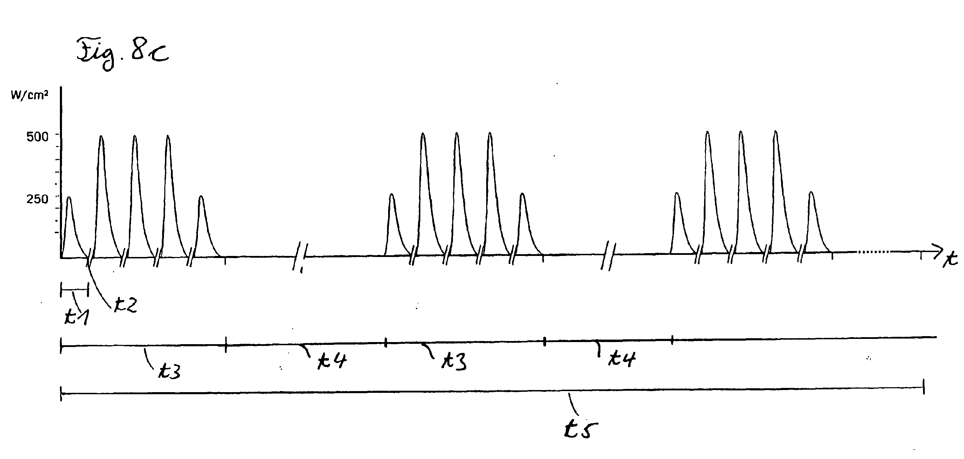

In der Fig. 8c ist letztlich die Kombination zwischen der Scan-Bewegung mit Pulsen dargestellt, wobei die Bestrahlungspeaks zwischen 250 und 500 W/cm² liegen. Dabei werden während der Zeit t3, wo die Bestrahlungsquelle ein bestimmtes Gebiet überstreicht, vorzugsweise 5 Pulse erzeugt, wobei der erste und der letzte Puls nur noch teilweise aufgrund der Bewegung das Gebiet erreichen. Die effektive Pulslänge wird vorzugweise mit 100 µs gewählt und die Bestrahlungsquelle mit 25 Hz getaktet, so daß sich t2 zu 40 ms bei einer Bestrahlungszeit t3 von 0,2 s ergibt. Jedoch sind die verschiedensten Kombinationen aus den Zahlenangaben der Ausführungsbeispiele nach den Fig.8a und b möglich.

Allgemein wurde beobachtet, dass die Beigabe von entzündungshemmenden Stoffen wie beispielsweise Cortison kontraproduktiv ist. Daher sollte auf die Einnahme derartiger Stoffe vor der Behandlung gänzlich verzichtet werden, wobei wegen der anhaltenden Wirkung vorzugsweise die entzündungshemmenden Stoffe einige Tage vorher abgesetzt werden sollten. Des weiteren wurde beobachtet, dass bei der Behandlung kleiner Flächen auch nicht behandelte Körperstellen abheilten. Dies deutet auf eine lokale systemische Wirkung der Bestrahlung hin, die ähnlich einer Immunisierung bei einer Impfung wirkt.

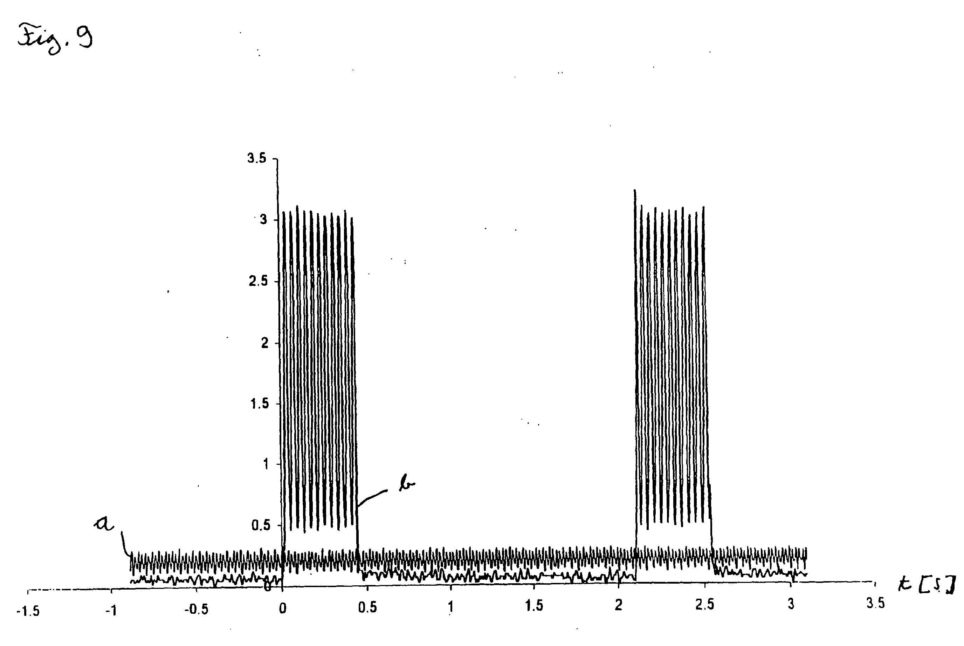

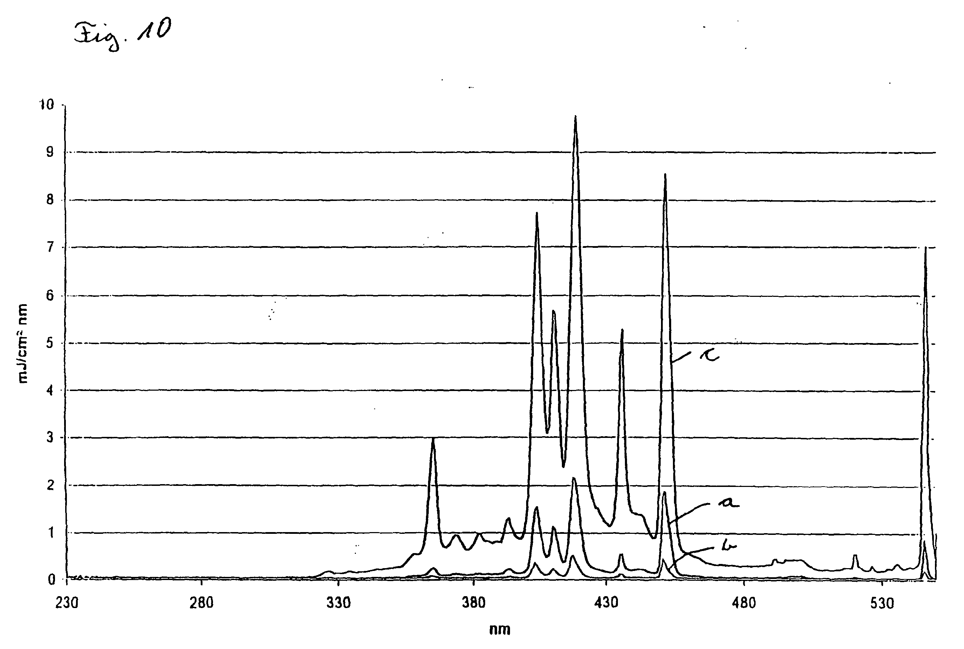

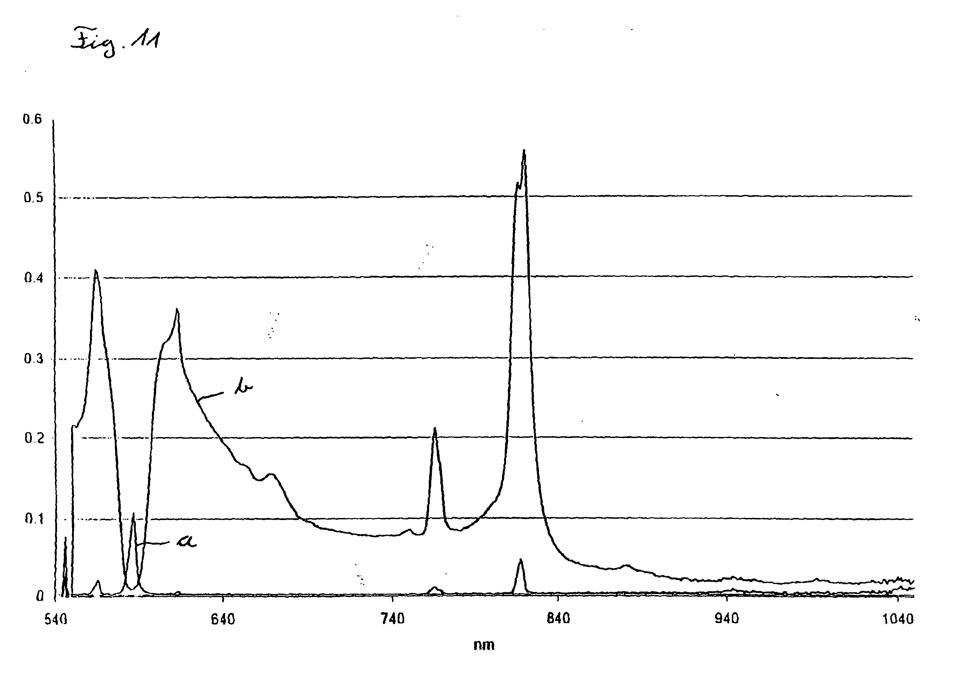

In der Fig.9 ist die relative Bestrahlungsstärke einer 1000 W Quecksilberiodid-Lampe im cw-Normalbetrieb bei 1000 W (Verlauf a) und im Pulsüberlastbetrieb (Verlauf b) dargestellt, wobei die gemittelte cw-Leistung im Pulsbetrieb 1500 W beträgt. Wie deutlich erkennbar, kommt es bereits bei geringer Überlast zu einer erheblichen Erhöhung der Emission. In der Fig.10 ist die spektrale Energiedichte bei einer derartigen galliumdotierten Quecksilberiodid-Lampe mit Nennleistung 1000 W dargestellt, wenn die Eingangsleistung variiert wird. Dabei stellt der Verlauf a) die Energiedichte im cw-Normalbetrieb bei 1000 W dar. Der Verlauf b) stellt die spektrale Energiedichte bei Unterlast von 100 W und der Verlauf c) stellt die Energiedichte bei 10 kW-Eingangsleistung dar, wobei hierbei sowohl Unter- als auch Überlast im cw-Betrieb betrieben wurden. Wie man jedoch deutlich erkennt, bleiben in beiden Fällen die Spektrallinien erhalten und es findet keine Inversion der Spektrallinien statt, sondern es kommt zu einer nahezu proportionalen Erhöhung der Emission. Im Gegensatz hierzu erkennt man deutlich in Fig.11 in Verlauf b) die Inversion der Spektrallinien bei einer Natriumdampflampe, die im Pulsbetrieb mit 700 W bei 230 W Nennleistung betrieben wird. Im Vergleich hierzu ist im Verlauf a) die relative Bestrahlungsstärke bei cw-Nennbetrieb dargestellt.

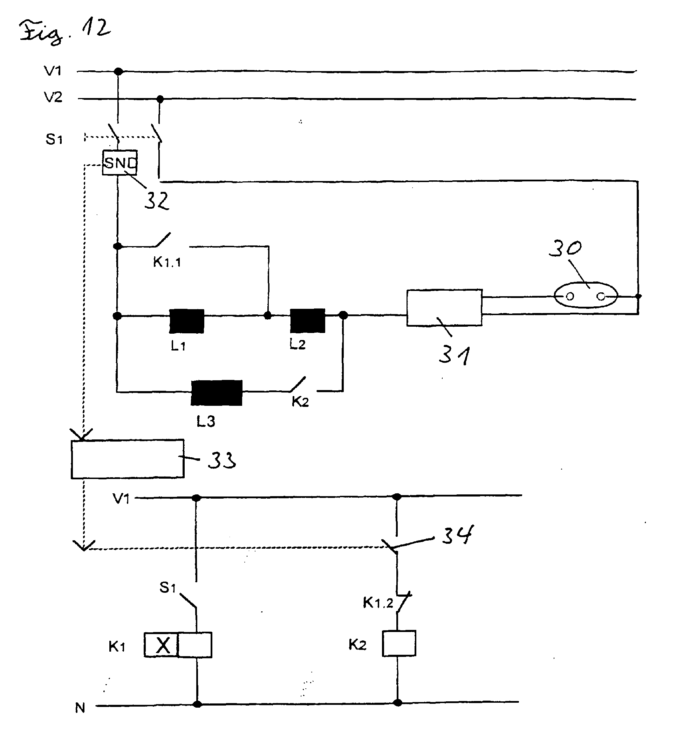

In der Fig.12 ist eine erste Schaltungsanordnung zum Betrieb einer galliumdotierten Quecksilberiodid-Lampe zum Pulsüberlastbetrieb dargestellt. Die Schaltung umfaßt eine galliumdotierte Quecksilberiodid-Lampe 30, einen Zündungsstab 31, einen Strom-Null-Durchgangsdetektor 32, einen Impulsgenerator 33, ein erstes Relais K1 und ein zweites Relais K2, sowie einen Starterschalter S1 und einen Impulsschalter 34. Die beiden Relais K1 und K2 sind beide mit einem Nullleiter N und einer ersten Phase V1 eines Drehstromnetzes verbunden. Die galliumdotierte Quecksilberiodid-Lampe 30 ist über einen Hilfskontakt des Starterschalters S1 mit einer zweiten Phase V2 des Drehstromnetzes verbunden. Über einen zweiten Hilfskontakt des Starterschalters S1 ist die erste Phase V1 über den Strom-Null-Durchgangsdetektor 32 über eine Spulenanordnung mit dem Zündungsstab 31 verbunden. Dabei sind die Spulen L1 und L2 in Reihe geschaltet. Parallel zu dieser Reihenschaltung ist eine dritte Spule L3 mit einem zum zweiten Relais K2 zugehörigen Kontakt K2 geschaltet. Parallel zur ersten Spule L1 ist ein erster zugehöriger Kontakt K1.1 des ersten Relais K1 geschaltet. Zwischen dem zweiten Relais K2 und dem Impulsschalter 34 ist ein zweiter zugehöriger Kontakt K1.2 des ersten Relais K1 geschaltet. Die prinzipielle Funktionsweise der Schaltung ist folgende:

Zuerst wird der Starterschalter S1 geschlossen, wodurch auch die beiden zugeordneten Hilfskontakte schliessen. Dadurch schließt der Kontakt K1.1 und der Kontakt K1.2 öffnet bzw. bleibt offen. Über den geschlossenen Kontakt K1.1 ist die erste Phase V1 des Drehstromnetzes über die Spule L2 mit dem Zündungsstab 31 verbunden, wobei die Spule L2 als Vorschaltdrossel wirkt. Dieser Schaltzustand bleibt solange erhalten, bis die galliumdotierte Quecksilberiodid-Lampe 30 ihre normalen Betriebsbedingungen erreicht hat. Anschließend fällt das Relais K1 ab, das beispielsweise als Einschaltwischer ausgebildet ist. Der Abfall von dem Relais K1 bewirkt ein öffnen des Kontaktes K1.1 und ein schließen des Kontaktes K1.2. Hierdurch wird das Relais K2 aktiviert und gleichzeitig die Spule L1 in Reihe zur Spule L2 geschaltet, wobei die Spule L1 als Simmerspule wirkt. Da der der Impulsschalter 34 noch offen ist, bleibt der Kontakt K2 noch offen. In diesem Zustand läuft die galliumdotierte Quecksilberiodid-Lampe 30 in einem Simmer. Zum Pulsbetrieb erfaßt nun der Strom-Null-Durchgangsdetektor 32 einen Nulldurchgang des Stromes der ersten Phase V1 des Drehstromnetzes und signalisiert dies dem Impulsgenerator 33. Dieser schaltet den Impulsschalter 34, so daß das Relais K2 bestromt wird und der Kontakt K2 geschlossen wird. Hierdurch wird die Spule L3 parallel geschaltet und die Gesamtinduktivität sinkt. wodurch der Zündungsstab 31 einen Überlastimpuls erhält. Am Ende des Pulses öffnet der Impulsgenerator 33 den Impulsschalter 34. Dadurch wird der Kontakt K2 geöffnet und die galliumdotierte Quecksilberiodid-Lampe 30 wird wieder im Simmer durch die Reihenschaltung der Spulen L1 und L2 betrieben, bis der nächste Impuls durch den Impulsgenerator 33 ausgelöst wird.

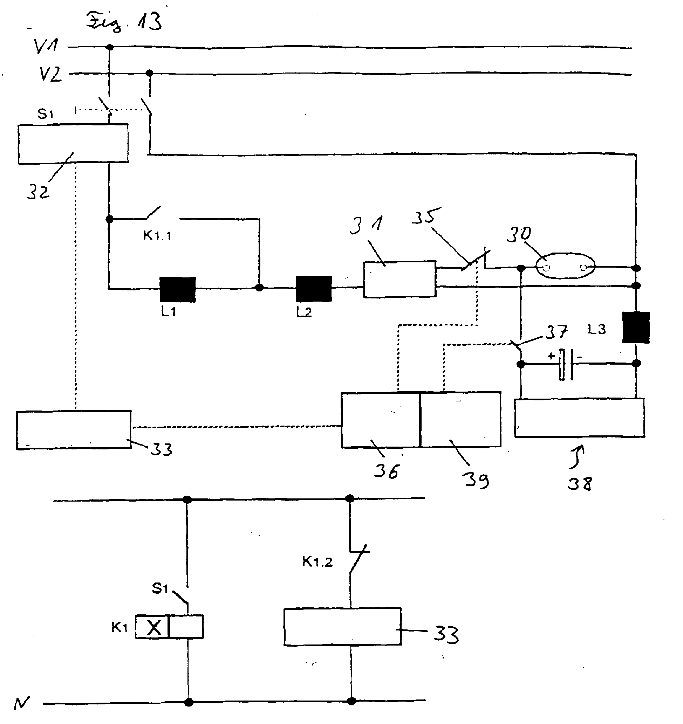

In der Fig.13 ist eine alternative Ausführungsform mit einer Kondensatorbank dargestellt, wobei gleiche Elemente in Bezug auf Fig.12 mit gleichen Bezugszeichen versehen sind. Im Unterschied zu der Ausführungsform gemäß Fig.12 ist zwischen dem Zündungsstab 31 und der galliumdotierten Quecksilberiodid-Lampe 30 ein Triac 35 angeordnet, dessen Treiber 36 vom Impulsgenerator 33 angesteuert wird. Über einen IGBT 37 bzw. die Spule L3 ist die Kondensatorbank 38 mit den Elektroden der galliumdotierten Quecksilberiodid-Lampe 30 verbunden, wobei der Treiber 39 des IGBT 37 ebenfalls von dem Impulsgenerator 33 angesteuert wird. Die Funktionsweise ist dabei wie folgt:
Wieder wird der Starterschalter S1 geschlossen, wodurch der Kontakt K1.1 schließt und der Kontakt K1.2 öffnet. Über den durchgeschalteten Triac 35 wird die galliumdotierte Quecksilberiodid-Lampe 30 auf Betriebsbedingungen hochgefahren. Anschließend fällt wieder das Relais K1 ab und der Kontakt K1.1 öffnet und K1.2 schließt. Die galliumdotierte Quecksilberiodid-Lampe 30 wird dann im Simmer über die Reihenschaltung der Spulen L1 und L2 betrieben und der Impulsgenerator 33 ist aktiviert. Für den Pulsbetrieb erfaßt dann der Strom-Null-Durchgangsdetektor 32 den Nulldurchgang und überträgt diese Information an den Impulsgenerator 33. Dieser steuert daraufhin die Treiber 36 und 39, so daß der Triac 35 sperrt und der IGBT 37 leitet. Dadurch wird die Kondensatorbank auf die galliumdotierte Quecksilberiodid-Lampe 30 geschaltet und diese von der Netzspannung getrennt. Am Ende des Pulses wird umgekehrt der IGBT 37 gesperrt und der Triac 35 durchgeschaltet., so daß die Lampe 30 wieder im Simmer über die beiden Spulen L1 und L2 läuft. Dabei sei angemerkt, daß wenn zuvor von Spulen die Rede war hierunter allgemein Induktivitäten zu verstehen sind. Zur Veranschaulichung der Größenverhältnisse dienen folgende Werte für die Spulen L1-L3: L1=500 mH; L2=150 mH und L3= 7 mH.
Pulsbetrieb: I_{eff}=40 A bzw. 11,8 A/cm², Iₚₑₐₖ=55 A bzw. 16,2 A/cm²;
Simmerbetrieb:I_{eff}=1,2 A bzw. 0,35 A/cm², Iₚₑₐₖ=1,7 A bzw. 0,5 A/cm²;
Normalbetrieb: I_{eff}=5 A bzw.1,5 A/cm², Iₚₑₐₖ=7 A bzw. 2 A/cm²;

## Patentansprüche

1. Bestrahlungsanordnung, insbesondere zur Behandlung von ganz oder teilweise zellvermittelten Entzündungen der Haut und der inneren Organe, umfassend mindestens eine Bestrahlungsquelle zur flächenhaften Bestrahlung einer Behandlungsfläche, wobei weniger als 7 % der optischen Gesamtleistung im UV-Bereich emittiert werden, und mindestens einen Spektralanteil im Wellenlängenbereich von 400 - 500 nm umfasst, wobei mindestens 30% der optischen Leistung in diesem Wellenlängenintervall emittiert werden;
wobei die Bestrahlungsanordnung zur Erzeugung von optischen Pulsen im Pulsbetrieb betreibbar und/oder relativ zu der behandelnden Fläche durch einen Scanner bewegbar ist, wobei, wenn die Bestrahlungsanordnung auf die Oberfläche aufgesetzt wird, die Bestrahlungsstärke der Bestrahlungspeaks der optischen Pulse größer 0,5 W/cm² und kleiner als 100 kW/cm² ist, wobei die Energiedichte eines emittierten optischen Pulses zwischen 0,3-0,8 J/cm² beträgt, wobei die Bestrahlungsquelle als Xe- oder Deuterium-Blitzlampe oder als im Pulsüberlast betriebene galliumdotierte Quecksilberiodid-Lampe ausgebildet ist, der eine Einrichtung zur Unterdrückung und/oder zur Transformierung der UV-Anteile und anderer nicht erwünschter Spektralanteile in dem gewünschten Spektralbereich zugeordnet ist.

2. Bestrahlungsanordnung, nach Anspruch 1, **dadurch gekennzeichnet, dass** die Pulse periodisch abgegeben werden, wobei die über die volle Periode gemittelte cw-Bestrahlungsstärke eines optischen Pulses zwischen 1 mW/cm²-10 W/cm² liegt.

3. Bestrahlungsanordnung nach Anspruch 1 oder 2, **dadurch gekennzeichnet, dass** die effektive Pulslänge zwischen 1 µs - 500 ms liegt.

4. Bestrahlungsanordnung nach Anspruch 3, **dadurch gekennzeichnet, dass** die Bestrahlungsquelle mit einer Frequenz von 0,01 -100 Hz getaktet ist.

5. Bestrahlungsanordnung nach einem der vorangegangenen Ansprüche, **dadurch gekennzeichnet, dass** vor der Bestrahlungsquelle eine Leuchtstoffschicht mit UVC-transparentem Trägermaterial angeordnet ist.

6. Bestrahlungsanordnung nach Anspruch 5, **dadurch gekennzeichnet, dass** das transparente Trägermaterial als Leuchtstofffolie (8) aus einem Silikonelastomer oder Fluorpolymer ausgebildet ist, die mit anorganischen Leuchtstoffpartikeln dotiert ist.

7. Bestrahlungsanordnung nach Anspruch 5 oder 6, **dadurch gekennzeichnet, dass** das transparente Trägermaterial oder die Leuchtstofffolie (8) mit mindestens einem der nachfolgenden Leuchtstoffpartikeln, fluoreszierend in den Spektralbereichen 410-490 nm
[Sr₂P₂ O₇ :Eu, Sr₅(PO₄)₃Cl:Eu, BaMg₂Al₁₆ O₂₇:Eu, Ca WO₄ : Pb; (Sr, Ca, Ba)₅(PO₄)₃Cl:Eu; Sr₂P₂O₇:Sn; (Ba, Ca)₅(PO₄)₃Cl:Eu)]
und/oder 510-560 nm
[ZnSiO₄:Mn; MgAl₁₁O₁₉:Ce,Tb,Mn; YBO₃:Tb; LaPO₄:Ce,Tb] und/oder 610-670 nm
[Y₂O₃:Eu; Y(P,V)O₄:Eu; CaSiO₃:Pb,Mn; (Sr,Mg)₃(PO₄)₂:Sn; 3.5MgO*0.5MgF₂*GeO₂:Mn]
dotiert ist.

8. Bestrahlungsanordnung nach einem der vorangegangenen Ansprüche, **dadurch gekennzeichnet, dass** der gepulsten Bestrahlungsquelle Mittel zugeordnet sind, mittels derer die Bestrahlungsquelle in einem Simmer betreibbar ist.

9. Bestrahlungsanordnung nach einem der vorangegangenen Ansprüche, **dadurch gekennzeichnet, dass** die Bestrahlungsanordnung eine Einrichtung zur Kühlung einer zu bestrahlenden Fläche und/oder der Bestrahlungsquelle und/oder der Leuchtstofffolie zugeordnet ist.

10. Bestrahlungsanordnung nach Anspruch 9, **dadurch gekennzeichnet, dass** die Einrichtung als Luftkühlung ausgebildet ist.

11. Bestrahlungsanordnung nach einem der vorangegangenen Ansprüche, **dadurch gekennzeichnet, dass** der Bestrahlungsanordnung Mittel zur topischen und/oder transpiratorischen Sauerstoffzufuhr zugeordnet sind.

12. Bestrahlungsanordnung nach einem der vorangegangenen Ansprüche, **dadurch gekennzeichnet, dass** die Bestrahlungsquelle zusätzlich im Bereich von 520 - 550 nm und/oder von 610 - 670 nm emittiert.

13. Bestrahlungsanordnung nach einem der vorangegangenen Ansprüche, **dadurch gekennzeichnet, dass** die Bestrahlungsquelle von einem Parabol-oder Ellipsoid-Reflektor umgeben ist.

14. Bestrahlungsanordnung nach einem der vorangegangenen Ansprüche, **dadurch gekennzeichnet, dass** der Strahlendurchmesser der emittierten Strahlung in mindestens einer Dimension größer 10 mm und kleiner 60 mm ist.

## Claims

1. Irradiation device, in particular for the treatment of totally or partially cell-mediated inflammations of the skin and of the internal organs, comprising at least one irradiation source for irradiation over a wide treatment area, wherein less than 7% of the total optical output is emitted in the UV range, and comprises at least one spectral component in the wavelength range of 400 -500 nm, wherein at least 30% of the optical output is emitted in this wavelength range;
wherein the irradiation device can be operated in pulse mode to generate optical pulses and/or can be moved relative to the treatment area by a scanner, wherein, when the irradiation device is placed on the surface, the intensity of irradiation of the irradiation peak of the optical pulses is greater than 0.5 W/cm² and less than 100 kW/cm², wherein the energy density of an emitted optical pulse is between 0.3 and 0.8 J/cm², wherein the irradiation source is in the form of a xenon or deuterium flash lamp or a gallium-doped mercury iodide lamp operated in pulse overload, equipped with a device for suppressing and/or transforming the UV components and other undesired components of the spectrum within the desired area of the spectrum.

2. Irradiation device according to claim 1, **characterised in that** the pulses are emitted periodically, wherein the average cw-irradiation intensity of one optical pulse over a complete period is between 1 mW/cm² 10 W/cm².

3. Irradiation device according to claim 1 or 2, **characterised in that** the effective pulse length is between 1 µs - 500 ms.

4. Irradiation device according to claim 3, **characterised in that** the irradiation source has a pulse frequency of 0.01 - 100 Hz.

5. Irradiation device according to one of the preceding claims, **characterised in that** a luminescent material layer with UVC-transparent carrier material is arranged in front of the irradiation source.

6. Irradiation device according to claim 5, **characterised in that** the transparent carrier material is in the form of a luminescent film (8) made from a silicone elastomer or fluoropolymer which is doped with inorganic luminescent particles.

7. Irradiation device according to claim 5 or 6, **characterised in that** the transparent carrier material or the luminescent film (8) is doped with at least one of the following luminescent particles, which are fluorescent in the spectral range of 410-490 nm:
[Sr₂P₂O₇: Eu, Sr₅(PO₄)₃Cl:Eu, BaMg₂Al₁₆ O₂₇:Eu, Ca WO₄: Pb; (Sr, Ca, Ba)₅(PO₄)₃Cl:Eu; Sr₂P₂O₇:Sn,(Ba, Ca)₅(PO₄)₃Cl:Eu)]
and/or 510-560 nm:
[ZnSiO₄:Mn; MgAl₁₁O₁₉:Ce,Tb,Mn; YBO₃:Tb; LaPO₄:Ce,Tb]
and/or 610-670 nm:
[Y₂O₃:Eu;Y(P,V)O₄:Eu; CaSiO₃:Pb,Mn; (Sr,Mg)₃(PO₄)₂:Sn; 3.5MgO*0.5MgF₂*GeO₂:Mn].

8. Irradiation device according to one of the preceding claims, **characterised in that** the pulsed irradiation source is equipped with means for operating said irradiation source in simmer mode.

9. Irradiation device according to one of the preceding claims, **characterised in that** the irradiation device is equipped with means of cooling an area to be irradiated and/or the irradiation source and/or the luminescent foil.

10. Irradiation device according to claim 9, **characterised in that** said means are designed as air cooling means.

11. Irradiation device according to one of the preceding claims, **characterised in that** the irradiation device is equipped with means for the topical and/or transpirational supply of oxygen.

12. Irradiation device according to one of the preceding claims, **characterised in that** the irradiation source additionally emits radiation in the range of 520 - 550 nm and/or 610 - 670 nm.

13. Irradiation device according to one of the preceding claims, **characterised in that** the irradiation source is enclosed by a paraboloidal or ellipsoidal reflector.

14. Irradiation device according to one of the preceding claims, **characterised in that** the beam diameter of the emitted radiation in at least one dimension is greater than 10 mm and less than 60 mm.

## Revendications

1. Système d'irradiation, notamment pour le traitement d'inflammations de la peau et des organes internes à médiation cellulaire totale ou partielle, comprenant au moins une source de rayonnement permettant d'irradier à plat une surface à traiter, moins de 7% de la puissance optique totale étant émise dans le domaine ultraviolet et comprenant au moins une composante spectrale dans la plage de longueur d'onde de 400 à 500 nm, au moins 30% de la puissance optique étant émise dans cet intervalle de longueur d'onde
lequel système d'irradiation peut être commandé pour générer des impulsions optiques en mode impulsionnel et/ou déplacé par rapport à la surface à traiter par un scanner, l'intensité d'irradiation au pic de rayonnement des impulsions optiques étant supérieure à 0,5 W/cm² et inférieure à 100 kW/cm² lorsque le système d'irradiation est posé sur la surface, l'impulsion optique émise ayant une densité d'énergie dans la plage de 0,3 à 0,8 J/cm², la source de rayonnement étant formée par une lampe à éclats au xénon ou au deutérium ou par une lampe à décharges à l'iodure de mercure dopé au gallium associée à un dispositif de suppression et/ou de transformation des composantes UV et d'autres composantes spectrales indésirables dans le domaine spectral voulu.

2. Système d'irradiation selon la revendication 1, **caractérisé en ce que** les impulsions sont délivrées de manière périodique, l'intensité d'irradiation cw d'une impulsion optique, moyennée sur la durée totale de la période, se situant dans la plage de 1 mW/cm² à 10 W/cm².

3. Système d'irradiation selon la revendication 1 ou la revendication 2, **caractérisé en ce que** la durée effective des impulsions va de 1 µs à 500 ms.

4. Système d'irradiation selon la revendication 3, **caractérisé en ce que** la source de rayonnement est cadencée à une fréquence de 0,01 à 100 Hz.

5. Système d'irradiation selon l'une des revendications précédentes, **caractérisé en ce qu'**une couche de matériau luminescent avec un matériau support transparent aux UVC est disposée avant la source de rayonnement.

6. Système d'irradiation selon la revendication 5, **caractérisé en ce que** le matériau support transparent servant de film luminescent (8) est constitué d'un élastomère de silicone ou d'un fluoropolymère dopé avec des particules de matériau luminescent inorganique.

7. Système d'irradiation selon la revendication 5 ou la revendication 6, **caractérisé en ce que** le matériau support transparent ou le film luminescent (8) est dopé avec au moins une particule des matériaux luminescents suivant émettant une fluorescence dans les domaines du spectre de 410 à 490 nm [Sr₂P₂O₇:Eu, Sr₅(PO₄)₃Cl:Eu, BaMg₂Al₁₆O₂₇:Eu, CaWO₄:Pb ; (Sr,Ca,Ba)₅(PO₄)₃Cl:Eu ; Sr₂P₂O₇:Sn ; (Ba,Ca)₅(PO₄)₃Cl:Eu)] et/ou de 510 à 560 nm [ZnSiO₄:Mn; MgAl₁₁O₁₉:Ce,Tb,Mn ; YBO₃:Tb ; LaPO₄:Ce,Tb] et/ou de 610 à 670 nm [Y₂O₃:Eu ; Y(P,V)O₄:Eu; CaSiO₃: Pb,Mn ; (Sr,Mg)₃(PO₄)₂:Sn ; 3,5MgO*0,5MgF₂*GeO₂:Mn].

8. Système d'irradiation selon l'une des revendications précédentes, **caractérisé en ce que** la source de rayonnement pulsée est associée à des moyens permettant de faire fonctionner la source de rayonnement en mode de pré-ionisation.

9. Système d'irradiation selon l'une des revendications précédentes, **caractérisé en ce que** le système d'irradiation est associé à un dispositif de refroidissement d'une surface à irradier et/ou de la source de rayonnement et/ou du film de matériau luminescent.

10. Système d'irradiation selon la revendication 9, **caractérisé en ce que** le dispositif est réalisé sous la forme d'un refroidissement par l'air.

11. Système d'irradiation selon l'une des revendications précédentes, **caractérisé en ce que** le système d'irradiation est associé à des moyens d'apport d'oxygène topique et/ ou transpiratoire.

12. Système d'irradiation selon l'une des revendications précédentes, **caractérisé en ce que** la source de rayonnement émet en plus dans la plage de 520 à 550 nm et/ ou de 610 à 670 nm.

13. Système d'irradiation selon l'une des revendications précédentes, **caractérisé en ce que** la source de rayonnement est entourée d'un réflecteur parabolique ou ellipsoïdal.

14. Système d'irradiation selon l'une des revendications précédentes, **caractérisé en ce que** le diamètre du faisceau du rayonnement émis est supérieur à 10 mm et inférieur à 60 mm dans au moins une dimension.
